(19) Europäisches Patentamt / European Patent Office / Office européen des brevets

(11) **EP 4 613 181 A1**

(12) **EUROPEAN PATENT APPLICATION**
published in accordance with Art. 153(4) EPC

(43) Date of publication:
**10.09.2025 Bulletin 2025/37**

(21) Application number: **23899910.6**

(22) Date of filing: **04.12.2023**

(51) International Patent Classification (IPC):
**A61B 5/01** (2006.01)    **A61B 5/024** (2006.01)
**A61B 5/318** (2021.01)    **A61B 5/389** (2021.01)
**A61B 5/0531** (2021.01)    **A61B 5/0205** (2006.01)

(52) Cooperative Patent Classification (CPC):
**A61B 5/02055; A61B 5/01; A61B 5/024;**
**A61B 5/02438;** A61B 5/0531; A61B 5/318;
A61B 5/389

(86) International application number:
**PCT/CN2023/136092**

(87) International publication number:
**WO 2024/120332 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **05.12.2022 CN 202211549724**

(71) Applicant: **Huawei Technologies Co., Ltd.
Shenzhen, Guangdong 518129 (CN)**

(72) Inventor: **CHEN, Baoyang
Shenzhen, Guangdong 518129 (CN)**

(74) Representative: **MERH-IP Matias Erny Reichl
Hoffmann
Patentanwälte PartG mbB
Paul-Heyse-Straße 29
80336 München (DE)**

(54) **WEARABLE DEVICE AND METHOD FOR IMPROVING ACCURACY OF PHYSIOLOGICAL INDEX MEASUREMENTS**

(57)  A wearable device is provided, and is configured to measure a physiological indicator of a user. **In the** wearable device, a thermal property measurement component is configured to measure a skin thermal property of an organism. The skin thermal property is used as a control signal to control to heat or cool skin of the organism. Because the skin thermal property of the organism is highly correlated with a blood perfusion rate and a skin water content that affect physiological indicator measurement accuracy, heating or cooling is controlled based on the thermal property, to effectively increase accuracy of a physiological indicator measurement component.

FIG. 3

**Description**

This application claims priority to Chinese Patent Application No. 202211549724.2, filed with the China National Intellectual Property Administration on December 5, 2022 and entitled "WEARABLE DEVICE AND METHOD FOR INCREASING PHYSIOLOGICAL INDICATOR MEASUREMENT ACCURACY", which is incorporated herein by reference in its entirety.

**TECHNICAL FIELD**

This application relates to the field of smart device technologies, and in particular, to a wearable device and a method for increasing physiological indicator measurement accuracy.

**BACKGROUND**

With advances in science and technology, wearable devices (Wearable Device) represented by a smart watch, a smart band, and a headset are purchased and used by broad consumers, and serve as one of manners of monitoring a physiological health indicator by the consumers. A heart rate, an electrocardiography, and an electromyography are most common measurement indicators in the wearable device. The wearable device is usually configured with a photoplethysmography (photoplethysmography, PPG) sensor, an electrocardiography (electrocardiography, ECG) sensor, and an electromyography (electromyography, EMG) sensor to implement measurement of the heart rate, the electrocardiography, the electromyography, and other indicators.

However, as an external environment changes, a physiological status of a human body easily changes. Consequently, the sensor configured on the wearable device faces a large measurement challenge. For example, in cold weather, a blood vessel of surface skin of the human body contracts, and a blood perfusion rate decreases, which easily causes a decrease in measurement accuracy of the PPG sensor. For another example, in dry weather, surface skin of the human body is dry, and it is difficult for the ECG sensor to have a high-quality connection to the human body, which also easily causes a decrease in measurement accuracy of the ECG sensor.

Therefore, currently, a method that can effectively increase measurement accuracy of a sensor on a wearable device is urgently needed.

**SUMMARY**

This application provides a wearable device. A thermal property measurement component is configured to measure a skin thermal property of an organism. The thermal property is used as a control signal to control to heat or cool skin of the organism, to change the skin thermal property, thereby increasing measurement accuracy of another component that has a correlation with the skin thermal property.

A first aspect of this application provides a wearable device, including a thermal property measurement component and a temperature regulation component.

The thermal property measurement component is configured to measure a skin thermal property of an organism. For example, the skin thermal property of the organism includes one or more of the following properties: thermal conductivity, thermal diffusivity, and thermal capacity.

The temperature regulation component is electrically connected to the thermal property measurement component, and is configured to control to heat or cool skin of the organism based on the skin thermal property of the organism, to change the skin thermal property of the organism. For example, when the skin thermal property of the organism indicates that a blood perfusion rate or a skin water content is low, the temperature regulation component may control to heat the skin of the organism. When the skin thermal property of the organism indicates that the skin water content is too high, the temperature regulation component may control to cool the skin of the organism. When the skin thermal property of the organism indicates that the blood perfusion rate or the skin water content meets a measurement requirement, the temperature regulation component may not perform temperature regulation on the skin of the organism.

In this solution, the thermal property measurement component is configured to measure the skin thermal property of the organism. The thermal property is used as a control signal to control to heat or cool the skin of the organism, to change the skin thermal property, thereby increasing measurement accuracy of another component that has a correlation with the skin thermal property, for example, increasing accuracy of a component that measures a heart rate or an electrocardiography.

In a possible implementation, the wearable device further includes a physiological indicator measurement component, configured to measure a physiological indicator of the organism. The skin thermal property of the organism is related to measurement accuracy of the physiological indicator measurement component. For example, the physiological indicator of the organism may be, for example, a heart rate, an electrocardiography, and/or an electromyography, and the physiological indicator measurement component may be, for example, a photoplethysmography (photoplethysmography, PPG) sensor, a bioelectrical impedance sensor, an electrocardiography (electrocardiography, ECG) sensor, an electromyography (electromyography, EMG) sensor, and/or a single heat flux sensor.

Because both a blood perfusion rate in the skin and a water content on a skin surface affect a transfer of heat on the skin of the organism (that is, affect the skin thermal property), the blood perfusion rate in the skin and the water content on the skin surface can be calculated based on a skin thermal property (namely, thermal conductivity, thermal diffusivity, and thermal capacity of the skin) that indicates a transfer of the heat on the skin of the organism. In this way, in this application, the blood perfusion rate in the skin and the water content on the skin surface can be learned by measuring the skin thermal property, to further determine whether heating or cooling needs to be performed on the skin, and change the skin thermal property of the organism, so that the blood perfusion rate in the skin and the water content on the skin surface meet a requirement, thereby ensuring measurement accuracy of the physiological indicator measurement component.

In addition, when the single heat flux sensor, or the like measures a temperature of deep tissue, a deep temperature is predicted based on a biothermal transfer equation and a temperature value of the temperature that is of the deep tissue and that is transferred to the skin surface. Therefore, measurement accuracy of measuring the temperature of the deep tissue by the single heat flux sensor can be further increased based on an accurate measured value (namely, the thermal conductivity, the thermal diffusivity, and the thermal capacity of the skin) of the skin thermal property that indicates the transfer of the heat on the skin of the organism.

In a possible implementation, the temperature regulation component includes a temperature regulation unit and a temperature control unit. The temperature regulation unit is configured to generate heat and/or absorb heat. The temperature control unit is configured to control, based on the skin thermal property of the organism, the temperature regulation unit to operate, to heat or cool the skin of the organism.

In a possible implementation, the temperature regulation unit includes one or more of the following components: a resistance wire, an electric heating plate, and a thermocouple.

In a possible implementation, the temperature regulation unit is built into a substrate of the wearable device, and the substrate of the wearable device is configured to be close to the skin of the organism.

Specifically, the substrate of the wearable device is in direct contact with human skin. Therefore, the temperature regulation unit is built into the substrate of the wearable device, so that the temperature regulation unit can be close to the human skin as much as possible, and the temperature regulation unit can well transfer heat by using the substrate. In addition, in this solution, the temperature regulation unit is built into the substrate, so that the temperature regulation unit can be prevented from being exposed outside the wearable device, and the temperature regulation unit is protected while a temperature regulating effect is ensured.

In a possible implementation, the physiological indicator measurement component and the temperature regulation unit are respectively built at different locations in the substrate.

In this solution, the physiological indicator measurement component and the temperature regulation unit are separately built at different locations in the substrate, so that measurement accuracy of the physiological indicator measurement component and a temperature regulation capability of the temperature regulation unit can be ensured, and mutual interference between the two components can be avoided.

In a possible implementation, the physiological indicator measurement component is disposed on a first surface of the substrate of the wearable device, the temperature regulation unit is built on the first surface of the substrate, a second surface of the substrate is configured to be close to the skin of the organism, and the first surface is parallel to the second surface.

In a possible implementation, the thermal property measurement component includes a thermal sensor and a heater, and the thermal sensor and the heater are configured to cooperate to operate, to measure the skin thermal property of the organism. The temperature regulation component is configured to control, based on the skin thermal property of the organism, the heater to heat the skin of the organism.

In this solution, the heater in the thermal property measurement component is used to regulate a skin temperature, so that no additional heater needs to be disposed on the wearable device, to reduce costs and reduce design complexity of the wearable device.

In a possible implementation, the physiological indicator measurement component includes the PPG sensor, and the PPG sensor is configured to measure the heart rate of the organism. The temperature regulation component is configured to control, based on the skin thermal property of the organism, a light emitter in the PPG sensor to continuously operate, to heat the skin of the organism.

In this solution, the light emitter in the PPG sensor is used to regulate the skin temperature, so that no additional heater needs to be disposed on the wearable device, to reduce costs and reduce design complexity of the wearable device.

In a possible implementation, the temperature regulation component is configured to control to heat the skin of the organism based on that a first skin thermal property of the organism is less than or equal to a first threshold. A first skin thermal property of a first organism may be any one of thermal conductivity, thermal diffusivity, and thermal capacity.

In this solution, a difference between the skin thermal property and a specific threshold is obtained through a comparison, to control whether to trigger to heat the skin. A control policy is simple, and measurement accuracy of the physiological indicator measurement component can be effectively increased, to improve implementability of the solution.

In a possible implementation, the temperature regulation component is further configured to control, based on a difference between the first skin thermal property of the organism and the first threshold, power and/or duration of heating the skin of the organism.

In this solution, the temperature regulation component regulates, based on a degree of a difference between the skin thermal property and the first threshold for triggering heating, power and/or duration of heating the skin, so that the skin can be accurately and effectively heated to a required state, to avoid repeatedly measuring the skin thermal property and heating, and improve skin heating efficiency.

In a possible implementation, the physiological indicator measurement component in the wearable device includes a PPG sensor; and the temperature regulation component is further configured to control to cool the skin of the organism based on that a second skin thermal property of the organism is greater than a second threshold. A first skin thermal property of a second organism may be any one of thermal conductivity, thermal diffusivity, and thermal capacity.

In a possible implementation, when the skin thermal property of the organism includes a plurality of properties, the temperature regulation component is configured to control to heat the skin of the organism based on a third threshold. The third threshold is a weighted summation result of the plurality of properties.

In this solution, whether to perform temperature regulation on the skin is determined by combining measurement results of the plurality of properties, so that reliability of controlling and regulating the skin temperature based on the skin thermal property can be improved, to increase accuracy of the physiological indicator measurement component and ensure user experience more reliably.

In a possible implementation, the temperature regulation component is further electrically connected to the physiological indicator measurement component; and the temperature regulation component is further configured to: obtain a signal measured by the physiological indicator measurement component, and control, based on quality of the signal measured by the physiological indicator measurement component, the thermal property measurement component to operate.

Specifically, when the quality of the signal measured by the physiological indicator measurement component is poor, it indicates that the blood perfusion rate or the water content of the skin may be low (in other words, a measurement requirement of the physiological indicator measurement component is not met). Therefore, the temperature regulation component may control the thermal property measurement component to operate, to measure a current skin thermal property. When the quality of the signal measured by the physiological indicator measurement component is high, it indicates that the blood perfusion rate or the skin water content is normal (that is, the measurement requirement of the physiological indicator measurement component is met). Therefore, the temperature regulation component no longer controls the thermal property measurement component to operate, to save energy consumption of the wearable device.

In a possible implementation, the temperature regulation component is further electrically connected to the physiological indicator measurement component; and the temperature regulation component is specifically configured to: obtain the signal measured by the physiological indicator measurement component, and heat or cool the skin of the organism based on the quality of the signal measured by the physiological indicator measurement component and the skin thermal property of the organism.

In this solution, the skin of the organism is controlled to be heated or cooled by combining quality of a physiological indicator measurement signal and the skin thermal property of the organism, so that a factor that affects measurement accuracy of the physiological indicator measurement component can be effectively determined, to effectively increase measurement accuracy of the physiological indicator measurement component and avoid affecting user experience as much as possible.

In a possible implementation, the wearable device further includes a single heat flux sensor, and the single heat flux sensor is configured to: measure a skin temperature of the organism, and determine a body temperature of the organism based on the skin temperature and the skin thermal property of the organism.

In a possible implementation, the wearable device is a watch, a band, a headset, a smart fabric, or a ring.

A second aspect of this application provides a method for increasing physiological indicator measurement accuracy. The method is applied to a wearable device, and includes: measuring a skin thermal property of an organism; and controlling to heat or cool skin of the organism based on the skin thermal property of the organism, to change the skin thermal property of the organism. The wearable device may include a physiological indicator measurement component, and the skin thermal property of the organism is related to measurement accuracy of the physiological indicator measurement component.

In a possible implementation, the skin thermal property of the organism includes one or more of the following properties: thermal conductivity, thermal diffusivity, and thermal capacity.

In a possible implementation, the skin thermal property of the organism may include only one property. Controlling to heat or cool the skin of the organism based on the skin thermal property of the organism specifically includes: controlling to heat the skin of the organism based on that a first skin thermal property of the organism is less than or equal to a first threshold.

In a possible implementation, in a process of controlling to heat the skin of the organism, power and/or duration of heating the skin of the organism are/is determined based on a difference between the skin thermal property of the organism and the first threshold.

In a possible implementation, the physiological indicator measurement component includes a PPG sensor. Controlling to heat or cool the skin of the organism based on the skin thermal property of the organism specifically includes: controlling to cool the skin of the organism based on that a second skin thermal property of the organism is greater than a second threshold.

In a possible implementation, when the skin thermal property of the organism includes a plurality of properties, controlling to heat or cool the skin of the organism based on the skin thermal property of the organism specifically includes: controlling to heat the skin of the organism based on a third threshold. The third threshold is obtained by performing weighted summation on the plurality of properties.

In a possible implementation, the method further includes: controlling, based on quality of a signal measured by the physiological indicator measurement component, a thermal property measurement component to operate.

In a possible implementation, the method further includes: controlling to heat or cool the skin of the organism based on quality of a signal measured by the physiological indicator measurement component and the skin thermal property of the organism.

In a possible implementation, the method further includes: obtaining a skin temperature of the organism, and determining a body temperature of the organism based on the skin temperature and the skin thermal property of the organism.

In a possible implementation, the physiological indicator measurement component includes one or more of the following sensors: a PPG sensor, a bioelectrical impedance sensor, an ECG sensor, an EMG sensor, and a single heat flux sensor.

In a possible implementation, the wearable device is a watch, a band, a headset, a smart fabric, or a ring.

A third aspect of this application provides a storage device, including a storage medium and a processor. The storage medium stores code, the processor is configured to execute the code, and when the code is executed, the storage device performs the method according to any implementation of the second aspect.

A fourth aspect of this application provides a computer storage medium. The computer storage medium stores instructions, and when the instructions are executed by a computer, the computer is enabled to implement the method according to any implementation of the second aspect.

A fifth aspect of this application provides a computer program product. The computer program product stores instructions, and when the instructions are executed by a computer, the computer is enabled to implement the method according to any implementation of the second aspect.

A sixth aspect of this application provides a chip. The chip includes a processor and a communication interface. The communication interface is configured to communicate with a module other than the chip. The processor is configured to run a computer program or instructions, so that an apparatus in which the chip is installed can perform the method according to any implementation of the second aspect.

For technical effects brought by any possible implementation of the second aspect to the sixth aspect, refer to technical effects brought by different implementations of the first aspect. Details are not described herein again.

## BRIEF DESCRIPTION OF DRAWINGS

FIG. 1 is a diagram of a measurement principle of a PPG sensor according to an embodiment of this application;
FIG. 2 is a diagram of measurement of an ECG sensor according to an embodiment of this application;
FIG. 3 is a diagram of a structure of a wearable device according to an embodiment of this application;
FIG. 4 is a diagram of a structure of a wearable device according to an embodiment of this application;
FIG. 5 is a diagram of deploying a component on a substrate of a wearable device according to an embodiment of this application;
FIG. 6 is a diagram of deploying a component on a substrate of a wearable device according to an embodiment of this application;
FIG. 7 is a diagram of an operating procedure of a wearable device according to an embodiment of this application;
FIG. 8 is a diagram of an operating procedure of another wearable device according to an embodiment of this application;
FIG. 9 is a diagram of an operating procedure of another wearable device according to an embodiment of this application;
FIG. 10 is a diagram of an operating procedure of another wearable device according to an embodiment of this application;
FIG. 11 is a diagram of a structure of a wearable device according to an embodiment of this application; and
FIG. 12 is a diagram of a structure of a computer-readable storage medium according to an embodiment of this application.

## DESCRIPTION OF EMBODIMENTS

The following describes embodiments of this application with reference to accompanying drawings. It is clear that the described embodiments are merely some rather than all of embodiments of this application. It can be learned by a person of ordinary skill in the art that, with development of a technology and emergence of a new scenario, the technical solutions provided in embodiments of this application are also applicable to similar technical problems.

In the specification, claims, and accompanying drawings of this application, the terms "first", "second", and so on are intended to distinguish between similar objects but do not necessarily indicate a specific order or sequence. It should be understood that the data termed in such a way are interchangeable in proper circumstances, so that embodiments described herein can be implemented in other orders than the order illustrated or described herein.

In addition, the terms "include" and "have" and any other variants thereof mean to cover the non-exclusive inclusion, for example, a process, method, system, product, or device that includes a list of steps or modules is not necessarily limited to those steps or modules, but may include other steps or modules not expressly listed or inherent to such a process, method, product, or device. Names or numbers of steps in this application do not mean that the steps in the method procedure need to be performed in a time/logical sequence indicated by the names or numbers. An execution sequence of the steps in the procedure that have been named or numbered can be changed based on a technical objective to be achieved, provided that same or similar technical effects can be achieved.

For ease of understanding, technical terms in embodiments of this application are first described below.

(1) Photoplethysmography (photoplethysmography, PPG) sensor

The PPG sensor is a sensor configured to measure a heart rate, and a principle of the PPG sensor is to measure the heart rate based on a difference in amounts of absorbed light corresponding to different blood volumes. Specifically, the PPG sensor includes a light emitter (for example a light-emitting diode (light-emitting diode, LED)) and a light receiver (for example, a photodetector (Photodetector, PD)). Because vascular pulsation caused by a heartbeat causes a change in a capillary blood volume at a location such as a wrist or an ear, detection light is emitted by using the LED, and a change in a light intensity signal received by the photodetector is analyzed, to obtain the heart rate through measurement.

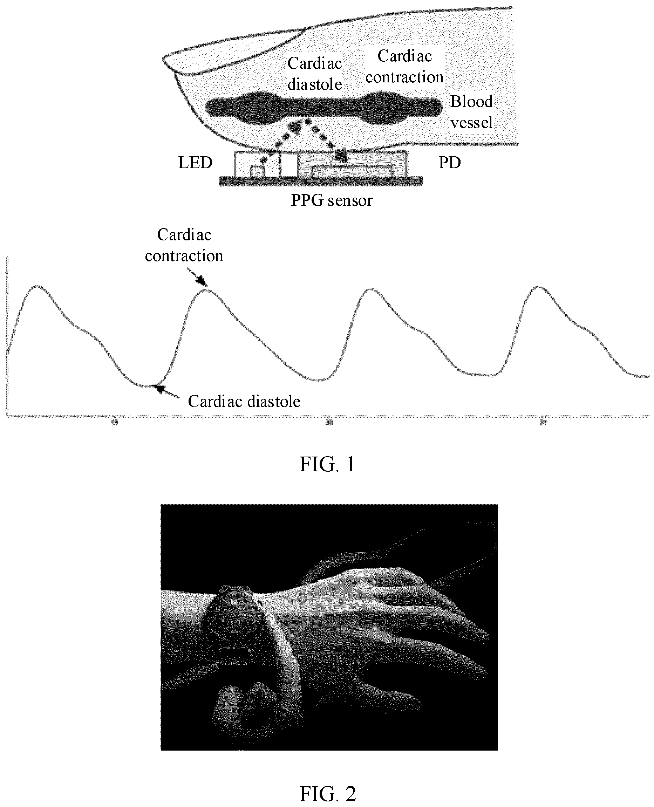

For example, FIG. 1 is a diagram of a measurement principle of a PPG sensor according to an embodiment of this application. As shown in FIG. 1, in the PPG sensor, the detection light emitted by the LED radiates a blood vessel in skin, and the PD receives a light intensity signal reflected by the blood vessel. When the light intensity signal shows a trough, it indicates that a heart is in a diastolic state; and when the light intensity signal shows a peak, it indicates that the heart is in a contraction state. Therefore, a specific value of the heart rate may be measured by measuring and recording the change in the light intensity signal.

(2) Bioelectrical impedance sensor

The bioelectrical impedance sensor is also a sensor used to measure a heart rate. A principle of the bioelectrical impedance sensor is to measure impedance of a biological body to monitor a blood flow, and convert the impedance into a specific heart rate, a specific respiratory rate, and a specific Galvanic skin response index.

(3) Electrocardiography (electrocardiography, ECG) sensor

The ECG sensor is a sensor used to measure an electrocardiography of a human body.

(4) Electromyography (electromyography, EMG) sensor

The EMG sensor is a sensor used to measure an electromyography of a human body.

Physiological electrical signals such as an electrocardiography and an electromyography are measured by using weak physiological electricity generated by a human muscle (including a cardiac muscle) during contraction and diastole. A potential difference between two or more electrodes in different parts of the body is measured, to capture different activity periods of the human muscle.

For example, FIG. 2 is a diagram of measurement of an ECG sensor according to an embodiment of this application. As shown in FIG. 2, an ECG sensor is deployed on a watch. An electrode of the ECG sensor is deployed on an inner side of a watch face of the watch, and is in contact with the back of a left hand of a user; and an electrode of the ECG sensor is deployed on an outer side of a watch bezel of the watch, and is in contact with a finger of a right hand of the user. In this way, a potential difference between electrodes in contact with different hands of a body of the user is measured, to measure an electrocardiography of the human body.

(5) Single heat flux sensor (single heat flux sensor)

The single heat flux sensor is an apparatus for measuring a heat flux passing through a surface of a sensor. In some application scenarios, the single heat flux sensor may be used to estimate a deep temperature of an attached object. Some researchers and companies use the single heat flux sensor to predict a deep skin temperature. The heat flux is heat energy that passes through a unit area per unit time.

(6) Thermal conductivity

The thermal conductivity, also referred to as a thermal conductance coefficient, is a physical quantity that indicates a thermal conductance capability of a material. A specific definition of the thermal conductivity is as follows: For two parallel planes with an area of 1 square meter and a distance of 1 meter in a direction perpendicular to a heat conduction direction in an object, if a temperature difference between the two planes is 1 K, heat conducted from one plane to the other plane within 1 second is specified as thermal conductivity of the material.

(7) Thermal diffusivity

In heat transfer analysis, thermal diffusivity a (unit: $m^2/s$) is a ratio of thermal conductivity $\lambda$ to a product of specific thermal capacity c and density $\rho$. The thermal diffusivity is also referred to as a thermal conductivity coefficient. The thermal diffusivity indicates an ability of an object to be heated or cooled at a uniform temperature, which is equivalent to a heat storage capacity of the object. The thermal conductivity indicates a speed at which an object conducts heat, and the thermal diffusivity is the ratio of the thermal conductivity $\lambda$ to the product of the specific thermal capacity c and the density $\rho$. Therefore, the thermal diffusivity reflects a thermal diffusivity capability of the object.

(8) Thermal capacity

A ratio of heat exchanged between a system and an environment when no phase change or chemical change occurs to a temperature change caused by this is referred to as thermal capacity of the system. Specifically, the thermal capacity is defined as heat absorbed (or released) by an object from an outside per increase (decrease) of a unit temperature.

(9) Thermocouple (thermocouple)

The thermocouple is a commonly used temperature measurement element in a temperature measurement instrument. The thermocouple directly measures a temperature, and converts a temperature signal into a thermoelectromotive force signal. The thermoelectromotive force signal is converted into a temperature of a measured medium by using an electrical instrument. Specifically, two ends of conductors (referred to as a thermocouple wire or thermoelectrode) with two different compositions are connected to form a loop. When temperatures of two connecting points are different, an electromotive force is generated in the loop. This phenomenon is referred to as a thermoelectric effect, and this electromotive force is referred to as a thermal electromotive force. The thermocouple is an element made based on the thermoelectric effect.

In general, the thermocouple is used to convert thermal energy into electrical energy.

(10) Signal-to-noise ratio (SIGNAL-NOISE RATIO, SNR)

The signal-to-noise ratio, also referred to as a signal-to-noise ratio, is a ratio of a signal to noise in an electronic device or an electronic system.

Currently, a heart rate, an electrocardiography, and an electromyography are most common measurement indicators in a wearable device. The wearable device usually implements measurement of indicators such as the heart rate, the electrocardiography, and the electromyography by being configured with a PPG sensor, an ECG sensor, and an EMG sensor.

However, due to complexity of a biological structure and a state of a human body and interference of an external environment, these three types of sensors face great measurement challenges.

An operating principle of the PPG sensor is to calculate a change in a blood volume based on a change of a light intensity signal, to calculate the heart rate. However, the light intensity signal received by the PPG sensor includes a direct current component (namely, a component that is of the signal and that does not change) generated by tissues such as skin, a vein, and ambient light and an alternating current component (namely, a component that is of the signal and that fluctuates) generated by vascular pulsation. The direct current component accounts for more than 90% of the light intensity signal, and the alternating current component accounts for only approximately 10% of the light intensity signal.

It can be learned from a composition of the light intensity signal that, a stronger arterial vessel activity indicates a larger total blood volume, a higher proportion of the alternating current component in the signal indicates a better parsed-out signal-to-noise ratio of the signal, and indicates that it is easier to parse out an accurate heart rate value. Therefore, the PPG sensor is more sensitive to a total blood volume at a measurement location, namely, a blood perfusion rate (perfusion index, PI). However, a location measured by the PPG is usually a peripheral vessel part such as a wrist or a finger, and a blood perfusion rate at the location is significantly affected by obesity and diabetes. In addition, because the peripheral vessel part such as the wrist or the finger is directly in contact with an external environment, a blood flow volume of an end such as a limb is reduced in a cold environment, and the PI is also significantly reduced.

In addition, for sensors that measure the electrocardiography and the electromyography such as the ECG sensor and the EMG sensor, an electrode needs to have a high-quality connection to a body during measurement. In other words, a proper contact resistance level is required, to provide a reliable signal with an enough amplitude for detection, and avoid too large signal attenuation. To ensure this, a wet electrode is usually used in a hospital. A most common wet electrode is a silver-/silver chloride (Ag/AgCl) electrode. However, in the wearable device, to ensure long-term reliability and an overall industrial design requirement, a metal dry electrode and a conductive ceramic dry electrode are usually selected. Contact conductivity between such a dry electrode and skin is reduced, and some moisture may still be required to be electrically coupled with a bioelectric potential generated in a physiological process that occurs in a body. A decrease in the contact conductivity makes a signal of a sensor have a poor overall signal-to-noise ratio and is more sensitive to a motion artifact.

It is found through research of the applicant that, in actual use, when more moisture penetrates between the skin and the electrode, conductivity between dry electrodes may increase. This is important for reducing contact impedance. Proper sweat can significantly improve a physiological electrical signal measurement effect. Because the sweat is rich in electrolyte, contact impedance between the skin and the dry electrode can be reduced. In addition, sweating skin has a higher water content, and impedance of the skin is smaller. For a physiological electrical signal at a millivolt level, a decrease in contact impedance and skin impedance caused by proper sweat can improve a measurement effect.

In general, in a measurement process of the PPG sensor, a skin temperature may be increased by heating the skin, and a blood vessel no longer contracts. Therefore, the blood perfusion rate is also increased accordingly, thereby increasing measurement accuracy of the PPG sensor. In measurement processes of the ECG sensor and the EMG sensor, a skin temperature may be increased by heating the skin, and sweat secretion is also increased accordingly, thereby increasing measurement accuracy of the ECG sensor and the EMG sensor. Therefore, in a specific scenario, measurement accuracy of the physiological indicator measurement component in the wearable device can be effectively increased by properly regulating the skin temperature.

However, in the conventional technology, it is not accurate enough to use the skin temperature as a threshold to trigger and disable heating. A low skin temperature cannot directly indicate a low blood perfusion rate and a low skin water content. When the blood perfusion rate and the skin water content are high enough, starting heating only increases energy consumption of the wearable device and affects user experience.

In view of this, an embodiment of this application provides a wearable device. A thermal property measurement component is configured to measure a skin thermal property of an organism. The thermal property is used as a control signal to control to heat or cool skin of the organism. Because the skin thermal property of the organism is highly correlated with a blood perfusion rate and a skin water content that affect physiological indicator measurement accuracy, heating or cooling is controlled based on the thermal property, to effectively increase accuracy of a physiological indicator measurement component.

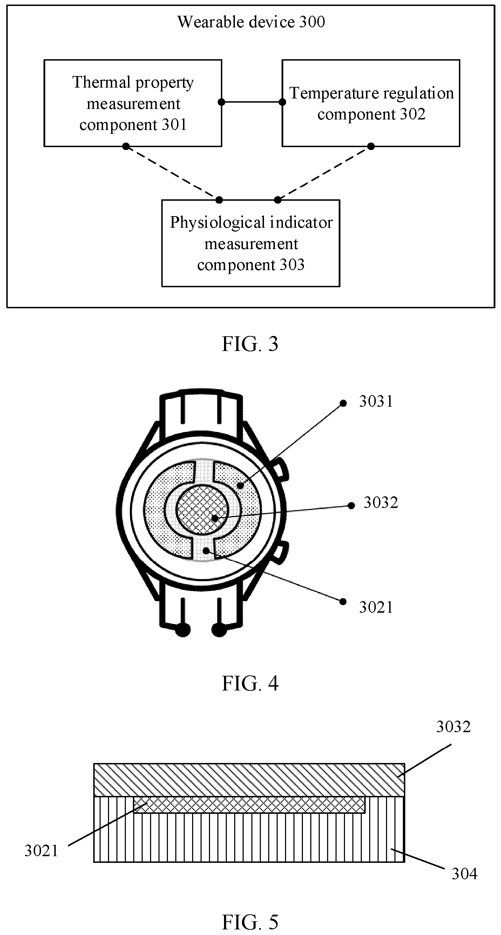

FIG. 3 is a diagram of a structure of a wearable device according to an embodiment of this application. As shown in FIG. 3, the wearable device 300 includes a thermal property measurement component 301, a temperature regulation component 302, and a physiological indicator measurement component 303. Optionally, the wearable device 300 is, for example, a watch, a band, a headset, a smart fabric, or a ring.

The thermal property measurement component 301 is configured to measure a skin thermal property of an organism. The organism described in this embodiment may be, for example, a human body or another animal. For ease of description, the following provides descriptions by using the human body as an example. The skin thermal property of the organism indicates a transfer of heat on skin of the organism. For example, the skin thermal property of the organism includes one or more of the following properties: thermal conductivity, thermal diffusivity, and thermal capacity. In other words, the thermal property measurement component 301 may be, for example, configured to measure thermal conductivity, thermal diffusivity, and/or thermal capacity of human skin. The thermal conductivity represents a speed of conducting heat by the skin; the thermal diffusivity represents a capability of diffusing heat by the skin; and the thermal capacity represents a relationship between a temperature change of the skin and heat.

The temperature regulation component 302 is electrically connected to the thermal property measurement component 301, and is configured to control to heat or cool the skin of the organism based on the skin thermal property of the organism, to change the skin thermal property of the organism.

The physiological indicator measurement component 303 is configured to measure a physiological indicator of the organism. The skin thermal property of the organism is related to measurement accuracy of the physiological indicator measurement component. For example, the physiological indicator of the organism may be, for example, a heart rate, an electrocardiography, and/or an electromyography, and the physiological indicator measurement component may be, for example, a PPG sensor, a bioelectrical impedance sensor, an ECG sensor, an EMG sensor, and/or a single heat flux sensor.

Logic of controlling and regulating a skin temperature by the temperature regulation component 302 is related to the physiological indicator measurement component 303, that is, an objective of controlling and regulating the skin temperature by the temperature regulation component 302 is to increase measurement accuracy of the physiological indicator measurement component 303. Therefore, the temperature regulation component 302 actually determines, based on the skin thermal property, whether a current skin state meets a measurement requirement of the physiological indicator measurement component 303, and adaptively regulates the skin temperature when the current skin state does not meet the measurement requirement of the physiological indicator measurement component 303, to increase measurement accuracy of the physiological indicator measurement component 303.

In brief, when the skin thermal property of the organism meets a first condition (for example, the skin thermal property of the organism indicates that a blood perfusion rate or a skin water content is low), the temperature regulation component 302 may control to heat the skin of the organism; or when the skin thermal property of the organism meets a second condition (for example, the skin thermal property of the organism indicates that a skin water content is too high), the temperature regulation component 302 may control to cool the skin of the organism; or when the skin thermal property of the organism meets a third condition (for example, the skin thermal property of the organism indicates that a blood perfusion rate or a skin water content meets the measurement requirement), the temperature regulation component 302 may not perform temperature regulation on the skin of the organism.

It should be noted that, when the physiological indicator measurement component 303 is a PPG sensor, because an operating principle of the PPG sensor is to measure a heart rate by transmitting and receiving an optical signal, and water has a strong absorption capability for an optical signal, when a water content on a skin surface is too high, normal receiving of the optical signal by the PPG sensor is also affected, affecting measurement accuracy of the PPG sensor. Therefore, when it is determined, based on the skin thermal property of the organism, that the skin water content is too high, the temperature regulation component 302 may control to cool the skin of the organism, to reduce sweat secretion, avoid a too high skin water content, and increase measurement accuracy of the physiological indicator measurement component 303.

Because both a blood perfusion rate in the skin and the water content on the skin surface affect a transfer of heat on the skin of the organism (that is, affect the skin thermal property), the blood perfusion rate in the skin and the water content on the skin surface can be calculated based on a skin thermal property (namely, thermal conductivity, thermal diffusivity, and thermal capacity of the skin) that indicates a transfer of the heat on the skin of the organism. In this way, in this application, the blood perfusion rate in the skin and the water content on the skin surface can be learned by measuring the skin thermal property, to further determine whether heating or cooling needs to be performed on the skin, and change the skin thermal property of the organism, so that the blood perfusion rate in the skin and the water content on the skin surface meet a requirement, thereby ensuring measurement accuracy of the physiological indicator measurement component.

In addition, when the single heat flux sensor, or the like measures a temperature of deep tissue, a deep temperature is predicted based on a biothermal transfer equation and a temperature value of the temperature that is of the deep tissue and that is transferred to the skin surface. Therefore, measurement accuracy of measuring the temperature of the deep tissue by the single heat flux sensor can be further increased based on an accurate measured value (namely, the thermal conductivity, the thermal diffusivity, and the thermal capacity of the skin) of the skin thermal property that indicates the transfer of the heat on the skin of the organism.

For ease of understanding, the following describes in detail components (namely, the thermal property measurement component, the temperature regulation component, and the physiological indicator measurement component) in the wearable device.

It can be learned from a definition of the skin thermal property that, the thermal property measurement component actually measures the transfer of the heat on the skin. Therefore, the thermal property measurement component needs to include an apparatus that can generate heat and an apparatus that measures the transfer of the heat on the skin. Specifically, in this application, the skin thermal property may be measured in different methods. For different measurement methods, the thermal property measurement component also has different structures.

For example, when the skin thermal property is measured in a direct current method, a measurement principle is specifically as follows: A current is supplied to a thermistor, so that the thermistor generates heat and the heat generated by the thermistor is conducted to a skin side. Then, in a specific time range (for example, 20 seconds), the thermistor and the skin reach a thermal balance, and temperature values of the thermistor at various time points are obtained, to obtain the thermal conductivity and the thermal diffusivity of the skin with reference to a formula.

Therefore, when the thermal property measurement component measures the skin thermal property in the direct current method, the thermal property measurement component includes at least one heater. The heater is, for example, a thermistor, configured to heat the skin. The thermal property measurement component may further include a thermal sensor, configured to measure the skin temperature, to determine the skin thermal property based on heat of heating the skin and a temperature change of the skin. Alternatively, the thermal property measurement component simultaneously implements functions of heating the skin and measuring the skin temperature by using the heater. For example, it is assumed that the heater is a thermal resistor including a resistance wire. When a large current is supplied to the resistance wire, the resistance wire generates joule heat, to heat the skin. When a constant current is supplied to the resistance wire and a voltage on both sides of the resistance wire is measured, or a constant voltage is supplied to the resistance wire and a current is measured, resistance of the resistance wire may be measured, and a temperature of the resistance wire is calculated based on a resistance temperature coefficient, to measure the skin temperature.

In addition, the thermal property measurement component may further include a processing module, configured to calculate the skin thermal property based on heat generated by the heater and a skin temperature measured by the temperature sensor in real time. Alternatively, the thermal property measurement component and the temperature regulation component may share one processing module. The processing module is configured to calculate the skin thermal property and control to heat or cool the skin based on the skin thermal property.

For example, when the skin thermal property is measured in a harmonic method, a measurement principle is specifically as follows: An alternating current signal is sent by a signal generator, and is applied to a heating wire after power is amplified by a power amplifier, so that the heating wire generates a heat wave to heat the skin. Then, a harmonic wave generated by the heating wire is measured, and after the harmonic wave is amplified, harmonic separation is performed by a lock-in amplifier, to calculate a temperature change. Finally, the thermal conductivity and thermal diffusivity of the skin are calculated based on a relationship between a heat wave frequency and a temperature. Therefore, when the thermal property measurement component measures the skin thermal property in the harmonic method, the thermal property measurement component may include apparatuses such as the signal generator, the power amplifier, the heating wire, an operational amplifier, and the lock-in amplifier.

The foregoing describes a possible structure of the thermal property measurement component when the skin thermal property is measured in the direct current method and the harmonic method. In addition to the foregoing structure, the thermal property measurement component may alternatively be of another structure to measure the skin thermal property. This is not specifically limited in this embodiment.

In this embodiment, the structure of the temperature regulation component may have a plurality of implementations.

Implementation 1: The temperature regulation component includes an apparatus that can regulate the skin temperature and an apparatus that determines, based on the skin thermal property, whether the skin temperature needs to be regulated.

For example, the temperature regulation component includes a temperature regulation unit and a temperature control unit. The temperature regulation unit is configured to generate heat and/or absorb heat. When the temperature regulation unit generates heat, the skin can be heated; and when the temperature regulation unit absorbs heat, the skin can be cooled. The temperature control unit is configured to control, based on the skin thermal property of the organism, the temperature regulation unit to operate, to heat or cool the skin of the organism.

Optionally, the temperature regulation unit includes one or more of the following components: a resistance wire, an electric heating plate, and a thermocouple. For the resistance wire and the electric heating sheet, a current is supplied to the resistance wire and the electric heating sheet, so that the resistance wire and the electric heating sheet can generate heat, to heat the skin. For the thermocouple, because the thermocouple can convert heat energy into electric energy, based on a principle of the thermocouple, the thermocouple may be used to absorb heat on the skin surface, to cool the skin.

In addition, when the temperature regulation unit is a resistance wire, an electric heating plate, or a thermocouple, in addition to heating or cooling the skin, the temperature regulation unit can further measure the skin temperature, to implement a temperature sensing function. For a principle of implementing temperature measurement by using the resistance wire, the electric heating plate, or the thermocouple, refer to the foregoing descriptions. Details are not described herein again.

Optionally, because the temperature regulation unit needs to directly heat or cool the skin, the temperature regulation unit may be disposed as close as possible to the skin. For example, the temperature regulation unit is built into a substrate of the wearable device, and the substrate of the wearable device is configured to be close to the skin of the organism. Specifically, the substrate of the wearable device is in direct contact with human skin. Therefore, the temperature regulation unit is built into the substrate of the wearable device, so that the temperature regulation unit can be close to the human skin as much as possible, and the temperature regulation unit can well transfer heat by using the substrate. In addition, the temperature regulation unit is built into the substrate, so that the temperature regulation unit can be prevented from being exposed outside the wearable device, and the temperature regulation unit is protected while a temperature regulating effect is ensured.

Optionally, because the physiological indicator measurement component also needs to be close to the human skin to measure a physiological indicator of a human body, the physiological indicator measurement component may also be built into the substrate of the wearable device. In addition, to avoid mutual interference between components, the physiological indicator measurement component and the temperature regulation unit are respectively built at different locations in the substrate.

For example, FIG. 4 is a diagram of a structure of a wearable device according to an embodiment of this application. As shown in FIG. 4, for example, the wearable device is a watch. An ECG sensor 3031, a PPG sensor 3032, and a temperature regulation unit 3021 are respectively built at different locations on a rear cover of the watch. Both the ECG sensor 3031 and the PPG 3032 are physiological indicator measurement components, and are respectively configured to measure an electrocardiography and a heart rate. The temperature regulation unit 3021 is a temperature regulation component, for example, a resistance wire, and is configured to heat the skin. Specifically, the ECG sensor 3031 may include two arc-shaped electrodes, which are respectively deployed on two sides of the rear cover of the watch, and are configured to measure an electrocardiography of the human body. The PPG sensor 3032 is deployed at a circular location in the center of the rear cover of the watch, and specifically includes an LED and a PD. The temperature regulation unit 3021 includes a resistance wire, and the resistance wire is disposed between the ECG sensor 3031 and the PPG sensor 3032.

In a possible implementation, the physiological indicator measurement component is disposed on a first surface of the substrate of the wearable device, the temperature regulation unit is built on the first surface of the substrate, a second surface of the substrate is configured to be close to the skin of the organism, and the first surface is parallel to the second surface. In other words, a back side of the substrate of the wearable device is directly in contact with the skin of the human body, and the temperature regulation unit and the physiological indicator measurement component are deployed on a front side of the substrate of the wearable device, so that the substrate of the wearable device provides protection for the temperature regulation unit and the physiological indicator measurement component.

For example, FIG. 5 is a diagram of deploying a component on a substrate of a wearable device according to an embodiment of this application. FIG. 5 shows a manner in which the PPG sensor 3032 and the temperature regulation unit 3021 are built on the substrate 304 of the wearable device. Specifically, for example, the substrate 304 of the wearable device is the rear cover of the watch. The substrate 304 of the wearable device is, for example, a sapphire, and a mesh resistance wire is disposed above the substrate 304 in a magnetron sputtering manner. The mesh resistance wire is the temperature regulation unit 3021, and a material of the resistance wire is, for example, a metal material with good conductivity, for example, platinum Pt, gold Au, or copper Cu. In addition, the PPG sensor 3032 may be further deployed above the substrate 304 of the wearable device, so that the substrate 304 of the wearable device protects the temperature regulation unit 3021 and the PPG sensor 3032.

The foregoing describes examples of deploying the temperature regulation component and the physiological indicator measurement component on the wearable device. The following describes specific examples of deploying the thermal property measurement component, the temperature regulation component, and the physiological indicator measurement component on the wearable device.

In a possible implementation, the thermal property measurement component includes a first heater and a thermal sensor, and the temperature regulation component includes a second heater and a temperature control unit. The physiological indicator measurement component includes, for example, a PPG sensor, an ECG sensor, and/or an EMG sensor. The first heater is configured to heat the skin, the thermal sensor is configured to detect the skin temperature, and the first heater is adjacent to the thermal sensor, that is, the first heater and the thermal sensor are deployed at adjacent locations. In this way, in a process in which the first heater heats the skin, the thermal sensor can quickly and effectively detect a temperature change that is of the skin and that is caused by heating by the first heater, to calculate the skin thermal property.

In addition, the second heater in the temperature regulation component may be deployed near the physiological indicator measurement component, and is configured to heat the skin in an area near the physiological indicator measurement component, to increase measurement accuracy of the physiological indicator measurement component. In addition, compared with the first heater, the second heater has a larger heating range, that is, the second heater has a larger deployment area on the wearable device, so that a blood perfusion rate in the skin can be effectively changed and the skin can be stimulated to secrete more sweat, thereby effectively increasing measurement accuracy of the physiological indicator measurement component.

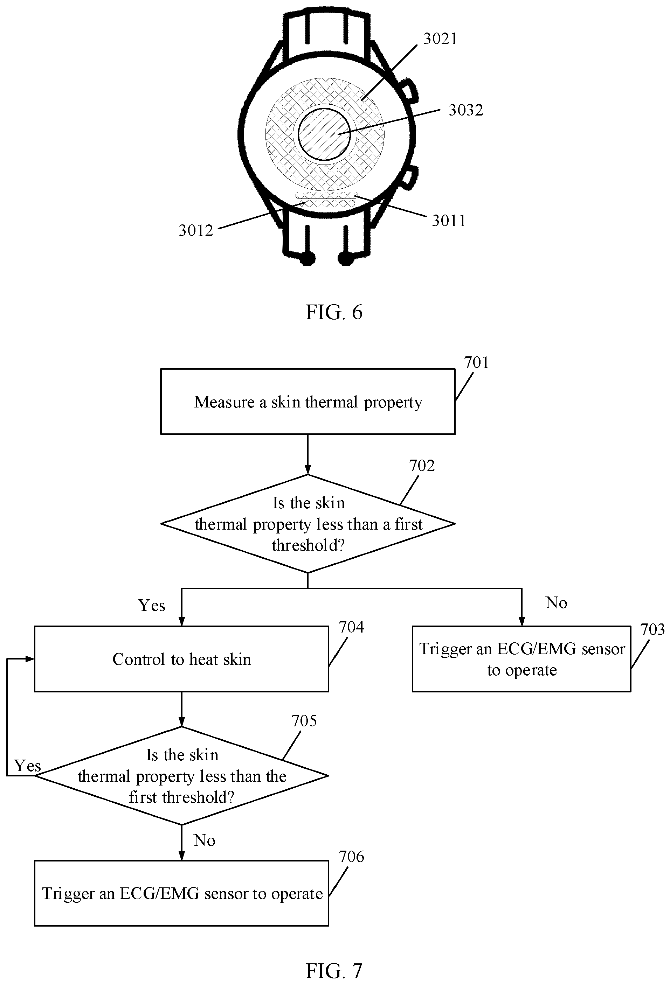

For example, FIG. 6 is a diagram of deploying a component on a substrate of a wearable device according to an embodiment of this application. As shown in FIG. 6, for example, the wearable device is a watch. A first heater 3011, a thermal sensor 3012, a temperature regulation unit 3021, and a PPG sensor 3032 are respectively built at different locations on a rear cover of the watch. The first heater 3011 and the thermal sensor 3012 are thermal property measurement components, and are configured to cooperate to measure the skin thermal property. The temperature regulation unit 3021 is a temperature regulation component, for example, a second heater, and is configured to heat the skin. The PPG sensor 3032 is a physiological indicator measurement component, and is configured to measure a heart rate.

Specifically, both the first heater 3011 and the thermal sensor 3012 may include resistance wires, and are respectively configured to perform heating and temperature measurement on the skin in a small range, to calculate the skin thermal property. The PPG sensor 3032 is deployed at a circular location in the center of the rear cover of the watch, and specifically includes an LED and a PD. The temperature regulation unit 3021 may be a second heater including a resistance wire. The second heater is in a ring shape, and the second heater is disposed on an outer side of the PPG sensor 3032, to effectively heat the skin in a large range outside the PPG sensor 3032, thereby increasing the blood perfusion rate, and increase measurement accuracy of the PPG sensor 3032.

Implementation 2: The temperature regulation component includes only an apparatus that determines, based on the skin thermal property, whether the skin temperature needs to be regulated.

In Implementation 2, the temperature regulation component is only configured to determine, based on the skin thermal property measured by the thermal property measurement component, whether the skin temperature needs to be regulated, and the thermal property measurement component or the physiological indicator measurement component heats the skin. Specifically, the temperature regulation component does not include an apparatus for regulating the skin temperature.

In a possible example, the thermal property measurement component may specifically include a thermal sensor and a heater, and the thermal sensor and the heater are configured to cooperate to operate, to measure the skin thermal property of the organism. The temperature regulation component is configured to control, based on the skin thermal property of the organism, the heater in the thermal property measurement component to heat the skin of the organism.

Specifically, in an operating process of the thermal property measurement component, the heater heats the skin, and the thermal sensor is configured to measure a temperature change that occurs when the skin is heated by the heater, to calculate the skin thermal property based on the temperature change state of the skin. When the temperature regulation component determines, based on the measured skin thermal property, that the skin needs to be heated, the heater may be controlled to continue to heat the skin. In addition, to quickly increase the skin temperature, the temperature regulation component may control an increase in an operating power of the heater, to increase a heat amount of the heater, thereby quickly increasing the blood perfusion rate in the skin or stimulating sweat secretion.

In this solution, the heater in the thermal property measurement component is used to regulate a skin temperature, so that no additional heater needs to be disposed on the wearable device, to reduce costs and reduce design complexity of the wearable device.

In another possible example, the physiological indicator measurement component includes a PPG sensor. The PPG sensor includes a light emitter (for example, an LED) and a light receiver (for example, a PD), and is configured to measure the heart rate of the organism. The temperature regulation component is configured to control, based on the skin thermal property of the organism, the light emitter in the PPG sensor to continuously operate, to heat the skin of the organism.

Specifically, the temperature regulation component may heat the skin based on a photothermal effect. The photothermal effect means that after an object is irradiated by light, photon energy interacts with a lattice, vibration is intensified, and a temperature is increased. In this way, the temperature regulation component controls an increase in a power of the light emitter in the PPG sensor, so that the light emitter continuously emits high-strength light energy, thereby increasing the skin temperature. In addition, the light emitter in the PPG sensor continuously operates at a high power, which also generates heat and transfers the heat to the skin, so that the skin can absorb the heat and heat up.

In this solution, the light emitter in the PPG sensor is used to regulate the skin temperature, so that no additional heater needs to be disposed on the wearable device, to reduce costs and reduce design complexity of the wearable device.

The foregoing describes a structure and a deployment manner of each component in the wearable device. The following specifically describes an operating principle of the temperature regulation component.

In a possible implementation, for a skin thermal property measured by a thermal property measurement component, the skin thermal property includes only one property. For example, the thermal property measurement component is only configured to measure thermal conductivity, thermal diffusivity, or thermal capacity. In this case, the temperature regulation component may be configured to control to heat skin of an organism based on that a skin thermal property of the organism is less than or equal to a first threshold.

It may be understood that, for any skin thermal property such as the thermal conductivity, the thermal diffusivity, or the thermal capacity, a higher skin thermal property indicates a stronger heat transfer capability of the skin and a higher blood perfusion rate and a higher water content of the skin. Therefore, measurement accuracy of the physiological indicator measurement component is also higher. A lower skin thermal property indicates a lower skin heat transfer capability and a lower blood perfusion rate and a lower water content of the skin. Therefore, measurement accuracy of the physiological indicator measurement component is also lower. To increase measurement accuracy of the physiological indicator measurement component, when the skin thermal property is lower than a specific threshold (namely, the first threshold), the skin may start to be heated, to increase the blood perfusion rate and the water content of the skin, thereby increasing measurement accuracy of the physiological indicator measurement component.

In this solution, a difference between the skin thermal property and a specific threshold is obtained through a comparison, to control whether to trigger to heat the skin. A control policy is simple, and measurement accuracy of the physiological indicator measurement component can be effectively increased, to improve implementability of the solution.

It may be understood that, for different types of physiological indicator measurement components, different physiological indicator measurement components may correspond to different conditions for triggering heating/cooling. Therefore, a value of the first threshold used to trigger the temperature regulation component to control to heat the skin may be determined based on a type of the physiological indicator measurement component.

For example, when the physiological indicator measurement component is a PPG sensor, measurement accuracy of the PPG sensor is mainly affected by the blood perfusion rate, and the blood perfusion rate decreases to a low value only in extremely cold weather. Therefore, heating may be triggered only when the skin thermal property is low. In other words, the first threshold for triggering the temperature regulation component to control to heat the skin may be set to a low value. For another example, when the physiological indicator measurement component is an ECG sensor, measurement accuracy of the ECG sensor is mainly affected by the skin water content, and the skin water content is increased to a high value only when the skin temperature is high. Therefore, heating is triggered when the skin thermal property is low. In other words, the first threshold for triggering the temperature regulation component to control to heat the skin may be set to a high value.

In other words, in this embodiment, a specific value of the first threshold may be determined based on a specific measurement principle of the physiological indicator measurement component. It should be noted that when the physiological indicator measurement component includes a plurality of measurement apparatuses, for example, the physiological indicator measurement component includes a PPG sensor and an ECG sensor, the value of the first threshold may be regulated in a targeted manner by combining a measurement requirement of the PPG sensor and a measurement requirement of the ECG sensor.

In addition, the value of the first threshold may also be comprehensively determined by balancing measurement accuracy of the physiological indicator measurement component and user experience. The specific value of the first threshold is not limited herein.

In brief, a smaller value of the first threshold indicates that the temperature regulation component is more likely to trigger to heat the skin, and therefore, measurement accuracy of the physiological indicator measurement component is higher. However, endurance of the wearable device is also lower, and a user more frequently perceives that the skin is heated, that is, user experience is lower. A larger value of the first threshold indicates that the temperature regulation component is less likely to trigger to heat the skin, and therefore, measurement accuracy of the physiological indicator measurement component is lower. However, endurance of the wearable device is also higher, and the user less frequently perceives that the skin is heated, that is, user experience is higher.

Optionally, the temperature regulation component is further configured to control, based on a difference between the skin thermal property of the organism and the first threshold, power and/or duration of heating the skin of the organism.

For example, a larger difference between a skin thermal property of a human body and the first threshold indicates a lower blood perfusion rate and a lower water content of human skin. Therefore, more heat needs to be transferred to the human skin, to increase a blood perfusion rate and a water content of the human skin as much as possible and control power and/or duration of heating the human skin to be greater. In this case, the temperature regulation component may control power of heating the skin of the organism to be a high power value; or the temperature regulation component may control duration of heating the skin of the organism to be a large duration value; or the temperature regulation component may control power of heating the skin of the organism to be a high power value and duration of heating the skin of the organism to be a large duration value.

For another example, a smaller difference between a skin thermal property of a human body and the first threshold indicates that a blood perfusion rate and a water content of the human skin are closer to a condition that needs to be met in measurement. Therefore, less heat needs to be transferred to the human skin, that is, power and/or duration of heating the human skin are/is controlled to be smaller. In this case, the temperature regulation component may control power of heating the skin of the organism to be a low power value; or the temperature regulation component may control duration of heating the skin of the organism to be a smaller duration value; or the temperature regulation component may control power of heating the skin of the organism to be a low power value and duration of heating the skin of the organism to be a small duration value.

In this solution, the temperature regulation component regulates, based on a degree of a difference between the skin thermal property and the first threshold for triggering heating, power and/or duration of heating the skin, so that the skin can be accurately and effectively heated to a required state, to avoid repeatedly measuring the skin thermal property and heating, and improve skin heating efficiency.

Optionally, when the physiological indicator measurement component includes a PPG sensor, the temperature regulation component is further configured to control to cool skin of the organism based on that the skin thermal property of the organism is greater than a second threshold. The second threshold is greater than the first threshold.

It may be understood that, because an operating principle of the PPG sensor is to measure a heart rate by transmitting and receiving an optical signal, and water has a strong absorption capability for an optical signal, when a water content on a skin surface is too high, normal receiving of the optical signal by the PPG sensor is also affected, affecting measurement accuracy of the PPG sensor. Therefore, when the skin thermal property is greater than the second threshold, the temperature regulation component may determine that the skin water content is too high, because the temperature regulation component may control to cool the skin of the organism, to reduce sweat secretion, avoid a too high skin water content, and increase measurement accuracy of the physiological indicator measurement component.

In addition, because a purpose of controlling cooling by the temperature regulation component is to reduce sweat secretion on the skin, and a sweat secretion amount is usually related to a sweat gland distribution on the skin, a value of the second threshold may be related to a location at which the wearable device is worn. When dense sweat glands are distributed at the location at which the wearable device is worn, the second threshold may be set to a low value. When sparse sweat glands are distributed at the location at which the wearable device is worn, the second threshold may be set to a high value.

In conclusion, when the physiological indicator measurement component includes the PPG sensor, measurement accuracy of the PPG sensor is the highest when the skin thermal property is in a specific range (namely, a threshold range between the first threshold and the second threshold); and measurement accuracy of the PPG sensor is low when the skin thermal property is less than the first threshold or greater than the second threshold.

It may be understood that, when the skin thermal property of the human body does not meet a measurement requirement, the blood perfusion rate and the water content of the human skin are low (or the skin water content is too high). Consequently, measurement accuracy of the physiological indicator measurement component is low. In this case, only when obtaining the skin thermal property that meets the measurement requirement, the wearable device may trigger the physiological indicator measurement component to operate, to prevent the physiological indicator measurement component from obtaining a low-quality physiological indicator signal through measurement. In other words, when the skin thermal property of the human body does not meet the measurement requirement, the physiological indicator measurement component is not triggered to operate, thereby effectively reducing energy consumption of the wearable device.

For example, FIG. 7 is a diagram of an operating procedure of a wearable device according to an embodiment of this application. As shown in FIG. 7, in the wearable device, a physiological indicator measurement component includes only an ECG sensor and/or an EMG sensor, and does not include a PPG sensor. The operating procedure of the wearable device includes steps 701 to 706.

Step 701: A thermal property measurement component in the wearable device measures a skin thermal property.

Specifically, the thermal property measurement component is triggered to measure the skin thermal property in a process in which the wearable device is normally worn by a user. For a specific process in which the thermal property measurement component measures the skin thermal property, refer to the descriptions in the foregoing embodiment. Details are not described herein again.

There may be a plurality of manners in which the thermal property measurement component is triggered to measure the skin thermal property. For example, the user delivers a thermal property measurement instruction to the wearable device by performing an operation on a display of the wearable device or directly operating a physical button of the wearable device, to trigger the thermal property measurement component to measure the skin thermal property. Alternatively, the user delivers a physiological indicator measurement instruction to the wearable device, to trigger the thermal property measurement component to measure the skin thermal property.

Step 702: A temperature regulation component in the wearable device determines whether the skin thermal property is less than a first threshold.

After the thermal property measurement component obtains the skin thermal property through measurement, the temperature regulation component in the wearable device compares a value relationship between the measured skin thermal property and the first threshold. If the skin thermal property is not less than the first threshold, step 703 is performed; or if the skin thermal property is less than the first threshold, step 704 is performed.

Step 703: If the skin thermal property is not less than the first threshold, the temperature regulation component triggers the ECG sensor and/or the EMG sensor to operate.

If the skin thermal property is not less than the first threshold, it indicates that a skin water content meets a measurement requirement of the ECG sensor and/or a measurement requirement of the EMG sensor. Therefore, the temperature regulation component may trigger the ECG sensor and/or the EMG sensor to operate, to obtain a physiological indicator of the user through measurement.

Step 704: If the skin thermal property is less than the first threshold, the temperature regulation component controls to heat skin.

If the skin thermal property is less than the first threshold, it indicates that the skin water content does not meet the measurement requirement of the ECG sensor and/or the measurement requirement of the EMG sensor. Therefore, the temperature regulation component may control to heat the skin, to increase the skin water content.

Step 705: After controlling to heat the skin, the temperature regulation component continues to determine whether a current skin thermal property is less than the first threshold.

If the current skin thermal property is still less than the first threshold, step 704 is performed to continue to heat the skin. If the current skin thermal property is not less than the first threshold, step 706 is performed to trigger the ECG sensor and/or the EMG sensor to operate.

Step 706: If the skin thermal property is not less than the first threshold, the temperature regulation component triggers the ECG sensor and/or the EMG sensor to operate.

In this solution, only when a skin thermal property of a human body meets a measurement requirement, the physiological indicator measurement component is triggered to operate, and when the skin thermal property of the human body does not meet the measurement requirement, the physiological indicator measurement component is not triggered to operate. In this way, the physiological indicator measurement component can be prevented from measuring an invalid physiological indicator, to effectively reduce energy consumption of the wearable device.

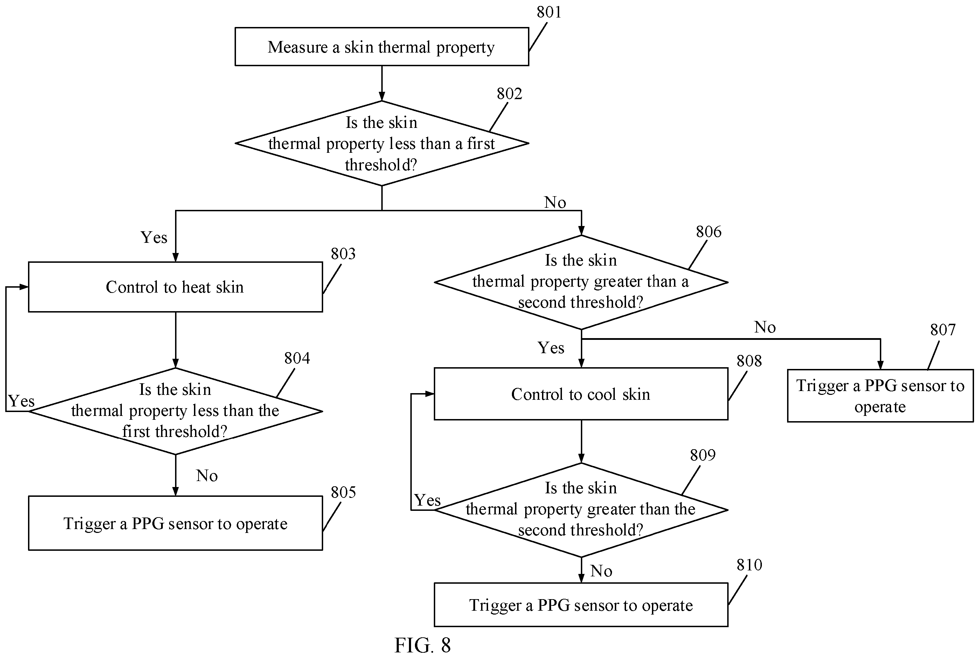

For example, FIG. 8 is a diagram of an operating procedure of another wearable device according to an embodiment of this application. As shown in FIG. 8, in the wearable device, a physiological indicator measurement component includes a PPG sensor. The operating procedure of the wearable device includes steps 801 to 810.

Step 801: A thermal property measurement component in the wearable device measures a skin thermal property.

Step 802: A temperature regulation component in the wearable device determines whether the skin thermal property is less than a first threshold.

After the thermal property measurement component obtains the skin thermal property through measurement, the temperature regulation component in the wearable device compares a value relationship between the measured skin thermal property and the first threshold. If the skin thermal property is less than the first threshold, step 803 is performed; or if the skin thermal property is not less than the first threshold, step 806 is performed.

It should be noted that the first threshold in FIG. 8 and the first threshold in FIG. 7 may have different values.

Step 803: If the skin thermal property is less than the first threshold, the temperature regulation component controls to heat skin.

If the skin thermal property is less than the first threshold, it indicates that a blood perfusion rate of skin does not meet a measurement requirement of the PPG sensor. Therefore, the temperature regulation component may control to heat the skin, to increase the blood perfusion rate of the skin.

Step 804: After controlling to heat the skin, the temperature regulation component continues to determine whether a current skin thermal property is less than the first threshold.

If the current skin thermal property is still less than the first threshold, step 803 is performed to continue to heat the skin. If the current skin thermal property is not less than the first threshold, step 805 is performed to trigger the PPG sensor to operate.

Step 805: If the current skin thermal property is not less than the first threshold, the temperature regulation component triggers the PPG sensor to operate.

Step 806: In a determining process of step 802, if the skin thermal property is not less than the first threshold, the temperature regulation component continues to determine whether the skin thermal property is greater than the second threshold.

If the skin thermal property is not greater than the second threshold, step 807 is performed to trigger the PPG sensor to operate; or if the skin thermal property is greater than the second threshold, step 808 is performed to control to cool the skin.

Step 807: If the skin thermal property is not greater than the second threshold, the temperature regulation component triggers the PPG sensor to operate.

If the skin thermal property is not greater than the second threshold, it indicates that the skin thermal property is between the first threshold and the second threshold, and meets the measurement requirement of the PPG sensor. Therefore, the temperature regulation component triggers the PPG sensor to operate.

Step 808: The temperature regulation component controls to cool the skin if the skin thermal property is greater than the second threshold.

If the skin thermal property is greater than the second threshold, it indicates that a current skin water content is too high, and measurement accuracy of the PPG sensor is easily affected. Therefore, the temperature regulation component controls to cool the skin, to reduce sweat secretion.

Step 809: After controlling to cool the skin, the temperature regulation component continues to determine whether the current skin thermal property is greater than the second threshold.

If the current skin thermal property is still greater than the second threshold, step 808 is performed to continue to cool the skin; or if the current skin thermal property is not greater than the second threshold, step 810 is performed to trigger the PPG sensor to operate.

Step 810: If the current skin thermal property is not greater than the second threshold, the temperature regulation component triggers the PPG sensor to operate.

The above describes controlling, based on a measurement result of a skin thermal property, a physiological indicator measurement component to operate. In some embodiments, a wearable device may alternatively control, based on a measurement result of a physiological indicator measurement component, a thermal property measurement component to operate.

For example, a temperature regulation component is further electrically connected to the physiological indicator measurement component. The temperature regulation component is further configured to: obtain a signal measured by the physiological indicator measurement component, and control, based on quality of the signal measured by the physiological indicator measurement component, the thermal property measurement component to operate.

Specifically, when the quality of the signal measured by the physiological indicator measurement component is poor, it indicates that a blood perfusion rate or a water content of the skin may be low (in other words, a measurement requirement of the physiological indicator measurement component is not met). Therefore, the temperature regulation component may control the thermal property measurement component to operate, to measure a current skin thermal property. When the quality of the signal measured by the physiological indicator measurement component is high, it indicates that the blood perfusion rate or the skin water content is normal (that is, the measurement requirement of the physiological indicator measurement component is met). Therefore, the temperature regulation component no longer controls the thermal property measurement component to operate, to save energy consumption of the wearable device.

Optionally, when the physiological indicator measurement component is a PPG sensor, a light intensity signal received by the PPG sensor includes an alternating current component related to the blood perfusion rate and a direct current component related to another factor. A larger alternating current component indicates a higher blood perfusion rate, and a smaller alternating current component indicates a lower blood perfusion rate. Therefore, in this embodiment, a ratio of the alternating current component to the direct current component in the light intensity signal received by the PPG may be used to measure quality of a physiological indicator signal measured by the PPG sensor. Specifically, a larger ratio of the alternating current component to the direct current component in the light intensity signal received by the PPG indicates a higher blood perfusion rate and higher quality of the physiological indicator signal measured by the PPG sensor; and a smaller ratio of the alternating current component to the direct current component in the light intensity signal received by the PPG indicates a lower blood perfusion rate and lower quality of the physiological indicator signal measured by the PPG sensor.

When the physiological indicator measurement component is an ECG sensor or an EMG sensor, because an electrocardiography signal or an electromyography signal measured by the ECG sensor or the EMG sensor is usually a continuous signal, a signal-to-noise ratio (namely, a ratio of a signal to noise) corresponding to the electrocardiography signal or the electromyography signal may be obtained as quality of the electrocardiography signal or the electromyography signal. A higher signal-to-noise ratio corresponding to the electrocardiography signal or the electromyography signal indicates less noise in the electrocardiography signal or the electromyography signal, and therefore higher quality of the electrocardiography signal or the electromyography signal. A lower signal-to-noise ratio corresponding to the electrocardiography signal or the electromyography signal indicates more noise in the electrocardiography signal or the electromyography signal, and therefore lower quality of the electrocardiography signal or the electromyography signal.

The foregoing describes controlling, based on a measurement result of a physiological indicator measurement component, a thermal property measurement component to operate. In some embodiments, a wearable device may further determine, based on a physiological indicator measurement result and a thermal property measurement result, whether to heat or cool skin.

For example, a temperature regulation component is further electrically connected to the physiological indicator measurement component. The temperature regulation component is specifically configured to: obtain a signal measured by the physiological indicator measurement component, and control to heat or cool skin of an organism based on quality of the signal measured by the physiological indicator measurement component and a skin thermal property of the organism.

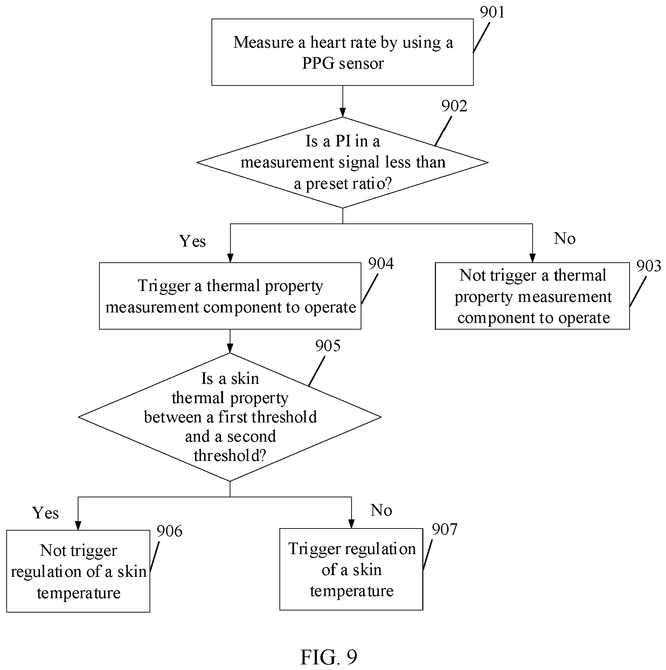

For example, FIG. 9 is a diagram of an operating procedure of another wearable device according to an embodiment of this application. As shown in FIG. 9, in the wearable device, a physiological indicator measurement component includes a PPG sensor. The operating procedure of the wearable device includes steps 901 to 907.

Step 901: The wearable device measures a heart rate by using the PPG sensor.

The wearable device may periodically trigger the PPG sensor to measure the heart rate. Alternatively, the wearable device may trigger, after obtaining an instruction delivered by a user, the PPG sensor to measure the heart rate.

Step 902: The wearable device determines whether a ratio (PI) of an alternating current component to a direct current component in a measurement signal of the PPG sensor is less than a preset ratio.

In an operating process of the PPG sensor, a light emitter in the PPG sensor emits light, and a light receiver receives a light intensity signal. The wearable device calculates the ratio (PI) of the alternating current component to the direct current component in the received light intensity signal, and determines whether the PI is less than a preset ratio. The preset ratio may be preset in the wearable device.

Step 903: If the PI in the measurement signal is not less than the preset ratio, the thermal property measurement component is not triggered to operate.

If the PI in the measurement signal is not less than the preset ratio, it indicates that a current blood perfusion rate of skin is high, and a measurement requirement of the PPG sensor is met. Therefore, the thermal property measurement component is not triggered to operate.

Step 904: If the PI in the measurement signal is less than the preset ratio, the thermal property measurement component is triggered to operate.

If the PI in the measurement signal is less than the preset ratio, it indicates that the current blood perfusion rate of the skin may be low, that is, the measurement requirement of the PPG sensor is not met. Therefore, the thermal property measurement component may be triggered to operate, to further determine whether the blood perfusion rate of the skin is low.

Step 905: Determine whether the measured skin thermal property is between a first threshold and a second threshold.

After the thermal property measurement component obtains the skin thermal property through measurement, the wearable device may further determine whether the measured skin thermal property is between the first threshold and the second threshold.

Step 906: Not trigger regulation of a skin temperature if the measured skin thermal property is between the first threshold and the second threshold.

If the measured skin thermal property is between the first threshold and the second threshold, it indicates that both the blood perfusion rate and the water content of the skin are at a normal level. In other words, a factor that affects measurement accuracy of the PPG sensor is not a too low blood perfusion rate of the skin or a too high skin water content, but another factor (for example, the wearable device is in poor contact with the skin or ambient light is too strong). In this case, measurement accuracy of the PPG sensor cannot be increased either by regulating the skin temperature, and therefore, regulating of the skin temperature is not triggered.

Step 907: Trigger regulation of the skin temperature if the measured skin thermal property is not between the first threshold and the second threshold.

If the measured skin thermal property is not between the first threshold and the second threshold, it indicates that the blood perfusion rate or the water content of the skin is not at the normal level. Therefore, the wearable device triggers regulation of the skin temperature. For example, the skin is triggered to be heated when the skin thermal property is less than the first threshold; or the skin is triggered to be cooled when the skin thermal property is greater than the second threshold.

In this solution, the skin of the organism is controlled to be heated or cooled by combining quality of a physiological indicator measurement signal and the skin thermal property of the organism, so that a factor that affects measurement accuracy of the physiological indicator measurement component can be effectively determined, to effectively increase measurement accuracy of the physiological indicator measurement component and avoid affecting user experience as much as possible.

In addition, in some embodiments, the wearable device may further determine, based on the skin temperature, the skin thermal property, and quality of a measured physiological indicator signal, whether to use a currently measured physiological indicator signal.

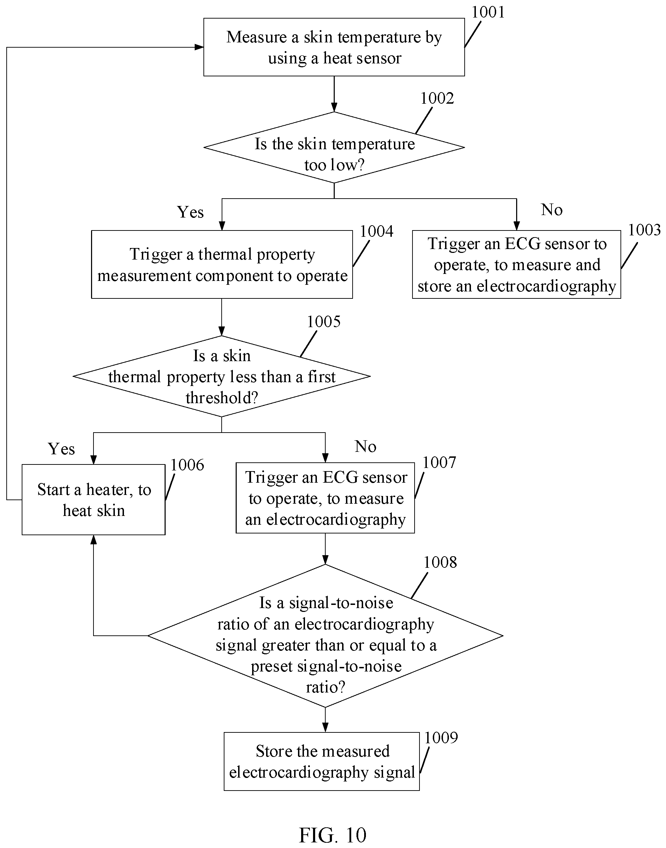

For example, FIG. 10 is a diagram of an operating procedure of another wearable device according to an embodiment of this application. As shown in FIG. 10, in the wearable device, a physiological indicator measurement component includes an ECG sensor. The operating procedure of the wearable device includes steps 1001 to 1009.

Step 1001: The wearable device measures a skin temperature by using a thermal sensor.

Step 1002: The wearable device determines whether the skin temperature is too low.

Step 1003: If the skin temperature is not low, the wearable device triggers the ECG sensor to operate, to measure and store an electrocardiography.

It may be understood that, if the skin temperature is not low, a skin water content is usually not low. In this case, the wearable device may trigger the ECG sensor to operate, to measure and store the electrocardiography.

Step 1004: If the skin temperature is too low, the wearable device triggers a thermal property measurement component to operate, to measure a skin thermal property.

If the skin temperature is too low, the skin water content may be too low. In this case, the wearable device may trigger the thermal property measurement component to operate, to measure the skin thermal property.

Step 1005: Determine whether the measured skin thermal property is less than a first threshold.

Step 1006: If the measured skin thermal property is less than the first threshold, control a start of a heater, to heat the skin.

If the measured skin thermal property is less than the first threshold, it indicates that the skin water content is low. Therefore, the wearable device controls a start of the heater, to heat the skin.

Step 1007: If the measured skin thermal property is not less than a first threshold, trigger the ECG sensor to operate, to measure an electrocardiography.

If the measured skin thermal property is not less than the first threshold, it indicates that the skin water content is not low, and therefore, the ECG sensor is triggered to operate, to measure the electrocardiography.

Step 1008: Determine whether a signal-to-noise ratio of a measured electrocardiography signal is greater than or equal to a preset signal-to-noise ratio.

If the signal-to-noise ratio of the measured electrocardiography signal is greater than or equal to the preset signal-to-noise ratio, it indicates that measurement accuracy of the ECG sensor is low. In this case, step 1006 may be performed to control a start of the heater, to heat the skin.

Step 1009: Store the measured electrocardiography signal if the signal-to-noise ratio of the measured electrocardiography signal is greater than or equal to the preset signal-to-noise ratio.

If the signal-to-noise ratio of the measured electrocardiography signal is not greater than the preset signal-to-noise ratio, it indicates that measurement accuracy of the ECG sensor meets a requirement. In this case, the measured electrocardiography signal can be stored.

In this solution, whether to further trigger the thermal property measurement component is first determined by measuring the skin temperature, so that when the skin temperature is high, the ECG sensor can be directly triggered to operate, and the thermal property measurement component does not need to be restarted to perform further measurement and determining, thereby effectively reducing power consumption of the wearable device.

The foregoing describes a case in which a wearable device determines, by measuring a skin thermal property, whether to perform temperature regulation on skin. In some embodiments, the wearable device may also determine, by measuring a plurality of skin thermal properties, whether to perform temperature regulation on the skin.

For example, a skin thermal property measured by a thermal property measurement component in the wearable device includes a plurality of properties. For example, the skin thermal property includes thermal conductivity and thermal diffusivity; or the skin thermal property includes thermal conductivity and thermal capacity; or the skin thermal property includes thermal conductivity, thermal diffusivity, and thermal capacity.

In this case, the temperature regulation component may be configured to control to heat skin of an organism based on a third threshold. The third threshold may be a weighted summation result of a plurality of properties. If the third threshold is less than or lower than a preset threshold, the skin of the organism is controlled to be heated.

Specifically, if the skin thermal property measured by the thermal property measurement component includes the thermal conductivity, the thermal diffusivity, and the thermal capacity, and the measured thermal conductivity is X1, the measured thermal diffusivity is X2, and the measured thermal capacity is X3, a weighted summation result of the plurality of properties is specifically a*X1+b*X2+c*X3. Herein, a+b+c=1; a, b, and c are all greater than 0 and less than 1.

In conclusion, in this solution, whether to perform temperature regulation on the skin is determined by combining measurement results of the plurality of properties, so that reliability of controlling and regulating the skin temperature based on the skin thermal property can be improved, to increase accuracy of the physiological indicator measurement component and ensure user experience more reliably.

For example, in a solution of measuring a temperature of a deep tissue in a single heat flux method, the temperature of the deep tissue may be measured based on temperatures of upper and lower layers of a single heat flux sensor and a thermal conductivity value.

$$T_{\text{core}} = T_{\text{bottom}}^{(i)} + g\left(T_{\text{bottom}}^{(i)} - T_{\text{top}}^{(i)}\right)$$

Herein, $T_{\text{core}}$ is the temperature of the deep tissue; $T_{\text{bottom}}^{(i)}$ is a temperature of the lower layer of the single heat flux sensor; $T_{\text{top}}^{(i)}$ is a temperature of the upper layer of the single heat flux sensor; $g = \dfrac{R_{\text{tissue}}}{R_{\text{partition}(i)}}$, $R_{\text{tissue}}$ is skin thermal resistance, $R_{\text{tissue}} \sim \dfrac{1}{\text{Thermal conductivity}}$, that is, the skin thermal resistance approximates to a reciprocal of skin thermal conductivity; and $R_{\text{partition}(i)}$ is thermal resistance of the single heat flux sensor. The lower layer of the single heat flux sensor is in direct contact with the skin of the organism, and the upper layer of the single heat flux sensor is located in the wearable device. Therefore, the temperature $T_{\text{bottom}}^{(i)}$ of the lower layer of the single heat flux sensor can be considered as a skin temperature of the organism. Heat on the skin of the organism may be first transferred to the lower layer of the single heat flux sensor in a direct contact manner, and then transferred to the upper layer of the single heat flux sensor.

It can be learned from the foregoing formula that, compared with an existing solution in which an empirical value is used as a skin thermal resistance, in this solution, skin thermal resistance is determined based on the measured skin thermal conductivity, and the temperature of the deep tissue is further calculated, so that measurement accuracy of the temperature of the deep tissue can be effectively increased.

Usually, the temperature that is of the deep tissue and that is measured by a single heat flux sensor can be considered as the body temperature of the organism. Therefore, the single heat flux sensor is actually used to measure the skin temperature of the organism and determine the body temperature of the organism based on the skin temperature of the organism and the skin thermal property.

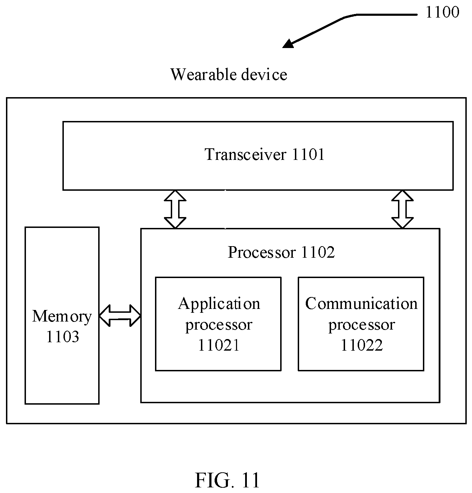

The following describes a wearable device according to an embodiment of this application. FIG. 11 is a diagram of a structure of a wearable device according to an embodiment of this application. The wearable device 1100 may be specifically represented as a watch, a band, a headset, a smart fabric, a ring, or the like. This is not limited herein. Specifically, the wearable device 1100 includes a transceiver 1101, a processor 1102, and a memory 1103 (there may be one or more processors 1102 in the wearable device 1100, and one processor is used as an example in FIG. 11). The processor 1102 may include an application processor 11021 and a communication processor 11022. In some embodiments of this application, the transceiver 1101, the processor 1102, and the memory 1103 may be connected through a bus or

The memory 1103 may include a read-only memory and a random access memory, and provide instructions and data to the processor 1102. Apart of the memory 1103 may further include a nonvolatile random access memory (nonvolatile random access memory, NVRAM). The memory 1103 stores a processor and operation instructions, an executable module or a data structure, a subnet thereof, or an extended set thereof. The operation instructions may include various operation instructions to implement various operations.

The processor 1102 controls an operation of the wearable device. In a specific application, the components of the wearable device are coupled together through a bus system. In addition to a data bus, the bus system may further include a power bus, a control bus, a status signal bus, and the like. However, for clear description, various types of buses in the figure are marked as the bus system.

The method disclosed in the foregoing embodiment of this application may be applied to the processor 1102, or implemented by the processor 1102. The processor 1102 may be an integrated circuit chip, and has a signal processing capability. In an implementation process, steps in the foregoing methods may be implemented by using a hardware integrated logic circuit in the processor 1102, or by using instructions in a form of software. The processor 1102 may be a general-purpose processor, a digital signal processor (digital signal processor, DSP), a microprocessor, or a microcontroller. The processor 1102 may further include an application-specific integrated circuit (application-specific integrated circuit, ASIC), a field programmable gate array (field programmable gate array, FPGA) or another programmable logic device, a discrete gate, or a transistor logic device, or a discrete hardware component. The processor 1102 may implement or perform the methods, the steps, and logical block diagrams that are disclosed in embodiments of this application. The general-purpose processor may be a microprocessor, or the processor may be any conventional processor or the like. The steps in the methods disclosed with reference to embodiments of this application may be directly performed and completed by a hardware decoding processor, or may be performed and completed by using a combination of hardware in the decoding processor and a software module. A software module may be located in a mature storage medium in the art, such as a random access memory, a flash memory, a read-only memory, a programmable read-only memory, an electrically erasable programmable memory, or a register. The storage medium is located in the memory 1103, and the processor 1102 reads information from the memory 1103 and accomplishes the steps in the foregoing methods in combination with the hardware of the processor 1102.

The transceiver 1101 (for example, a network adapter) may be configured to: receive input digital or character information, and generate a signal input related to a related setting and function control of the wearable device. The transceiver 1101 may be further configured to: output digit or character information through a first interface, and send instructions to a disk group through the first interface, to modify data in the disk group. The transceiver 1101 may further include a display device such as a display.

In this embodiment of this application, in one case, the processor 1102 is configured to perform the method performed by the wearable device in the embodiments corresponding to FIG. 7 to FIG. 10.

The wearable device provided in this embodiment of this application may specifically include a chip. The chip includes a processing unit and a communication unit. The processing unit may be, for example, a processor, and the communication unit may be, for example, an input/output interface, a pin, or a circuit. The processing unit may perform computer-executable instructions stored in a storage unit, so that the chip in the wearable device performs the method for increasing physiological indicator measurement accuracy described in the foregoing embodiments. Optionally, the storage unit is a storage unit in the chip, for example, a register or a cache. Alternatively, the storage unit may be a storage unit in a wireless access device but outside the chip, for example, a read-only memory (read-only memory, ROM), another type of static storage device that can store static information and instructions, or a random access memory (random access memory, RAM).

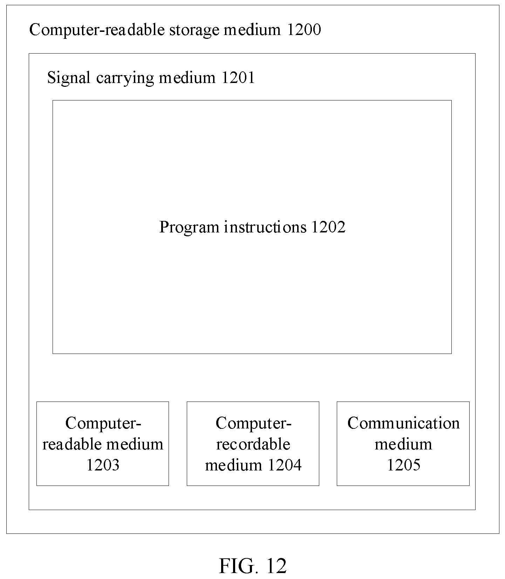

FIG. 12 is a diagram of a structure of a computer-readable storage medium according to an embodiment of this application. This application further provides a computer-readable storage medium. In some embodiments, the method disclosed in FIG. 7 to FIG. 10 may be implemented as computer program instructions encoded in a machine-readable format on the computer-readable storage medium or encoded on another non-transitory medium or product.

FIG. 12 schematically shows a conceptual partial view of an example computer-readable storage medium arranged according to at least some embodiments shown herein, including a computer program for executing a computer process on a computing device.

In an embodiment, the computer-readable storage medium 1200 is provided by using a signal carrying medium 1201. The signal carrying medium 1201 may include one or more program instructions 1202, and when being run by one or more processors, the program instructions 1202 may provide the foregoing functions or some functions described for FIG. 2. In addition, the program instructions 1202 in FIG. 12 alternatively describes an example instruction.

In some examples, the signal carrying medium 1201 may include a computer-readable medium 1203 such as but not limited to a hard disk drive, a compact disc (CD), a digital video disc (DVD), a digital tape, a memory, a ROM, or a RAM.

In some implementations, the signal carrying medium 1201 may include a computer-recordable medium 1204 such as but not limited to a memory, a read/write (R/W) CD, or an R/W DVD. In some implementations, the signal carrying medium 1201 may include a communication medium 1205 such as but not limited to a digital and/or analog communication medium (for example, an optical fiber cable, a waveguide, a wired communication link, or a wireless communication link). Therefore, for example, the signal carrying medium 1201 may be communicated by a wireless communication medium 1205 (for example, a wireless communication medium that complies with the IEEE 802.12 standard or another transmission protocol).

The one or more program instructions 1202 may be, for example, computer-executable instructions or logic implementation instructions. In some examples, a computing device may be configured to provide various operations, functions, or actions in response to the program instructions 1202 transmitted to the computing device by using one or more of the computer-readable medium 1203, the computer-recordable medium 1204, and/or the communication medium 1205.

In addition, it should be noted that the described apparatus embodiment is merely an example. The units described as separate parts may or may not be physically separate, and parts displayed as units may or may not be physical units, may be located at one location, or may be distributed on a plurality of network units. Some or all the modules may be selected according to actual needs to achieve the objectives of the solutions of embodiments. In addition, in the accompanying drawings of the apparatus embodiments provided by this application, connection relationships between modules indicate that the modules have communication connections with each other, which may be specifically implemented as one or more communication buses or signal cables.

Based on the description of the foregoing implementations, a person skilled in the art may clearly understand that this application may be implemented by software in addition to necessary universal hardware, or by dedicated hardware, including an application-specific integrated circuit, a dedicated CPU, a dedicated memory, a dedicated component, and the like. Usually, any functions that can be performed by a computer program can be easily implemented by using corresponding hardware. Moreover, a specific hardware structure used to achieve a same function may be in various forms, for example, in a form of an analog circuit, a digital circuit, or a dedicated circuit. However, as for this application, software program implementation is a better implementation in most cases. Based on such an understanding, the technical solutions of this application essentially or the part contributing to the conventional technology may be implemented in a form of a software product. The computer software product is stored in a readable storage medium, such as a floppy disk, a USB flash drive, a removable hard disk, a ROM, a RAM, a magnetic disk, or an optical disc of a computer, and includes several instructions for instructing a computer device (which may be a personal computer, a training device, a network device, or the like) to perform the methods in embodiments of this application.

All or some of the foregoing embodiments may be implemented by using software, hardware, firmware, or any combination thereof. When software is used to implement the embodiments, all or a part of the embodiments may be implemented in a form of a computer program product.

The computer program product includes one or more computer instructions. When the computer program instructions are loaded and executed on the computer, the procedure or functions according to embodiments of this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, a computer, a training device, or a data center to another website, computer, training device, or data center in a wired (for example, a coaxial cable, an optical fiber, or a digital subscriber line (DSL)) or wireless (for example, infrared, radio, or microwave) manner. The computer-readable storage medium may be any usable medium that can be stored by a computer, or a data storage device, such as a training device or a data center, integrating one or more usable media. The usable medium may be a magnetic medium (for example, a floppy disk, a hard disk, or a magnetic tape), an optical medium (for example, a DVD), a semiconductor medium (for example, a solid state disk (Solid State Disk, SSD)), or the like.

**Claims**

1. A wearable device, comprising:

   a thermal property measurement component, configured to measure a skin thermal property of an organism; and
   a temperature regulation component, wherein the temperature regulation component is electrically connected to the thermal property measurement component, and is configured to control to heat or cool skin of the organism based on the skin thermal property of the organism.

2. The wearable device according to claim 1, wherein the wearable device further comprises a physiological indicator measurement component, configured to measure a physiological indicator of the organism.

3. The wearable device according to claim 1 or 2, wherein the wearable device comprises a photoplethysmography PPG sensor, and the PPG sensor is configured to measure a heart rate of the organism; and

the temperature regulation component is configured to control, based on the skin thermal property of the organism, a light emitter in the PPG sensor to continuously operate, to heat the skin of the organism.

4. The wearable device according to any one of claims 1 to 3, wherein the skin thermal property of the organism comprises one or more of the following properties: thermal conductivity, thermal diffusivity, and thermal capacity.

5. The wearable device according to any one of claims 1 to 4, wherein
the temperature regulation component is configured to control to heat the skin of the organism based on that a first skin thermal property of the organism is less than or equal to a first threshold.

6. The wearable device according to any one of claims 1 to 5, wherein
the temperature regulation component is further configured to control to cool the skin of the organism based on that a second skin thermal property of the organism is greater than a second threshold.

7. The wearable device according to any one of claims 1 to 4, wherein when the skin thermal property of the organism comprises a plurality of properties,
the temperature regulation component is configured to control to heat the skin of the organism based on a third threshold, wherein the third threshold is obtained by performing weighted summation on the plurality of properties.

8. The wearable device according to any one of claims 1 to 7, wherein the wearable device further comprises the physiological indicator measurement component, and the temperature regulation component is further electrically connected to the physiological indicator measurement component; and
the temperature regulation component is further configured to control, based on quality of a signal measured by the physiological indicator measurement component, the thermal property measurement component to operate.

9. The wearable device according to any one of claims 1 to 7, wherein the wearable device further comprises the physiological indicator measurement component, and the temperature regulation component is further electrically connected to the physiological indicator measurement component; and
the temperature regulation component is specifically configured to heat or cool the skin of the organism based on quality of a signal measured by the physiological indicator measurement component and the skin thermal property of the organism.

10. The wearable device according to any one of claims 1 to 9, wherein the wearable device further comprises a single heat flux sensor, and the single heat flux sensor is configured to: measure a skin temperature of the organism, and determine a body temperature of the organism based on the skin temperature and the skin thermal property of the organism.

11. The wearable device according to any one of claims 1 to 10, wherein the temperature regulation component comprises a temperature regulation unit and a temperature control unit;

the temperature regulation unit is configured to generate heat and/or absorb heat; and
the temperature control unit is configured to control, based on the skin thermal property of the organism, the temperature regulation unit to operate, to heat or cool the skin of the organism.

12. The wearable device according to claim 11, wherein the temperature regulation unit is built into a substrate of the wearable device, and the substrate of the wearable device is configured to be close to the skin of the organism.

13. The wearable device according to claim 12, wherein the wearable device further comprises the physiological indicator measurement component, the physiological indicator measurement component is disposed on a first surface of the substrate of the wearable device, the temperature regulation unit is built on the first surface of the substrate, a second surface of the substrate is configured to be close to the skin of the organism, and the first surface is parallel to the second surface.

14. The wearable device according to any one of claims 1 to 10, wherein the thermal property measurement component comprises a thermal sensor and a heater, and the thermal sensor and the heater are configured to cooperate to operate, to measure the skin thermal property of the organism; and
the temperature regulation component is configured to control, based on the skin thermal property of the organism, the heater to heat the skin of the organism.

15. The wearable device according to any one of claims 1 to 14, wherein the wearable device further comprises the physiological indicator measurement component, and the physiological indicator measurement component comprises one or more of the following sensors: a PPG sensor, a bioelectrical impedance sensor, an electrocardiography ECG sensor, an electromyography EMG sensor, and a single heat flux sensor.

16. The wearable device according to any one of claims 1 to 15, wherein the wearable device is a watch, a band, a headset, a smart fabric, or a ring.

17. A method for increasing physiological indicator measurement accuracy, wherein the method is applied to a wearable device, and comprises:

obtaining a skin thermal property of an organism; and
controlling to heat or cool skin of the organism based on the skin thermal property of the organism.

18. The method according to claim 17, wherein the skin thermal property of the organism comprises one or more of the following properties: thermal conductivity, thermal diffusivity, and thermal capacity.

19. The method according to claim 17 or 18, wherein
the controlling to heat or cool the skin of the organism based on the skin thermal property of the organism comprises:
controlling to heat the skin of the organism based on that a first skin thermal property of the organism is less than or equal to a first threshold.

20. The method according to any one of claims 17 to 19, wherein the wearable device comprises a PPG sensor; and
the controlling to heat or cool the skin of the organism based on the skin thermal property of the organism comprises:
controlling to cool the skin of the organism based on that a second skin thermal property of the organism is greater than a second threshold.

21. The method according to claim 17 or 18, wherein when the skin thermal property of the organism comprises a plurality of properties,
the controlling to heat or cool the skin of the organism based on the skin thermal property of the organism comprises:
controlling to heat the skin of the organism based on a third threshold, wherein the third threshold is obtained by performing weighted summation on the plurality of properties.

22. The method according to any one of claims 17 to 21, wherein the method further comprises:
controlling, based on quality of a signal measured by the physiological indicator measurement component, the thermal property measurement component to operate.

23. The method according to any one of claims 17 to 21, wherein the method further comprises:
controlling to heat or cool the skin of the organism based on quality of a signal measured by the physiological indicator measurement component and the skin thermal property of the organism.

24. The method according to any one of claims 17 to 23, wherein the method further comprises: obtaining a skin temperature of the organism; and
determining a body temperature of the organism based on the skin temperature and the skin thermal property of the organism.

25. A computer storage medium, wherein the computer storage medium stores instructions, and when the instructions are executed by a computer, the computer is enabled to implement the method according to any one of claims 17 to 24.

26. A computer program product, wherein the computer program product stores instructions, and when the instructions are executed by a computer, the computer is enabled to implement the method according to any one of claims 17 to 24.

FIG. 1

FIG. 2

Wearable device 300

Thermal property
measurement
component 301

Temperature regulation
component 302

Physiological indicator
measurement
component 303

FIG. 3

3031

3032

3021

FIG. 4

3032

3021

304

FIG. 5

FIG. 6

Measure a skin thermal property 701

Is the skin thermal property less than a first threshold? 702

Yes

No

Control to heat skin 704

Trigger an ECG/EMG sensor to operate 703

Is the skin thermal property less than the first threshold? 705

Yes

No

Trigger an ECG/EMG sensor to operate 706

FIG. 7

801 Measure a skin thermal property

802 Is the skin thermal property less than a first threshold?

No →

Yes →

806 Is the skin thermal property greater than a second threshold?

No → 807 Trigger a PPG sensor to operate

Yes →

808 Control to cool skin

809 Is the skin thermal property greater than the second threshold?

Yes → (back to 808 Control to cool skin)

No → 810 Trigger a PPG sensor to operate

803 Control to heat skin

804 Is the skin thermal property less than the first threshold?

Yes → (back to 803 Control to heat skin)

No → 805 Trigger a PPG sensor to operate

FIG. 8

Measure a heart rate by using a PPG sensor — 901

Is a PI in a measurement signal less than a preset ratio? — 902

Yes

No

Trigger a thermal property measurement component to operate — 904

Not trigger a thermal property measurement component to operate — 903

Is a skin thermal property between a first threshold and a second threshold? — 905

Yes

No

Not trigger regulation of a skin temperature — 906

Trigger regulation of a skin temperature — 907

FIG. 9

```
                        ┌─────────────────────────┐  1001
                        │  Measure a skin temperature by │╱
                        │     using a heat sensor      │
                        └─────────────────────────┘
                                    │
                                    ▼
                              ╱───────────╲  1002
                            ╱ Is the skin temperature ╲╱
                            ╲       too low?          ╱
                              ╲───────────╱
                    Yes │                          │ No
                        ▼                          ▼
      ┌─────────────────────┐ 1004   ┌──────────────────────┐ 1003
      │  Trigger a thermal property  │╱      │ Trigger an ECG sensor to │╱
      │ measurement component to operate │      │   operate, to measure and │
      └─────────────────────┘        │ store an electrocardiography │
                    │                          └──────────────────────┘
                    ▼
              ╱───────────╲  1005
            ╱   Is a skin    ╲╱
            ╲ thermal property less than a first ╱
            ╲      threshold?   ╱
              ╲───────────╱
      Yes │                    │ No
          ▼                    ▼
 ┌──────────┐ 1006   ┌──────────────────┐ 1007
 │ Start a heater, to │╱      │ Trigger an ECG sensor │╱
 │    heat skin       │      │  to operate, to measure │
 └──────────┘        │   an electrocardiography │
                              └──────────────────┘
                                    │
                                    ▼
                              ╱───────────╲  1008
                            ╱  Is a signal-to-noise  ╲
                            ╲ ratio of an electrocardiography ╱
                            ╲ signal greater than or equal to a ╱
                            ╲ preset signal-to-noise ╱
                            ╲       ratio?     ╱
                              ╲───────────╱
                                    │
                                    ▼
                        ┌──────────────────┐ 1009
                        │   Store the measured  │╱
                        │ electrocardiography signal │
                        └──────────────────┘
```

FIG. 10

1100

Wearable device

| Transceiver 1101 |
| --- |

| Memory 1103 | Processor 1102 |
| --- | --- |
| | Application processor 11021 | Communication processor 11022 |

FIG. 11

Computer-readable storage medium 1200

Signal carrying medium 1201

Program instructions 1202

| Computer-readable medium 1203 | Computer-recordable medium 1204 | Communication medium 1205 |

FIG. 12

# INTERNATIONAL SEARCH REPORT

International application No.

**PCT/CN2023/136092**

### A. CLASSIFICATION OF SUBJECT MATTER

A61B5/01(2006.01)i; A61B5/024(2006.01)i; A61B5/318(2021.01)i; A61B5/389(2021.01)i; A61B5/0531(2021.01)i

According to International Patent Classification (IPC) or to both national classification and IPC

### B. FIELDS SEARCHED

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; ENTXTC; WPABSC; VEN; ENTXT; CNKI; 百度学术, BAIDU SCHOLAR; IEEE: 华为, 热属性, 热学属性, 热导率, 热导速率, 热导速度, 热传导率, 热传导速率, 热传导速度, 热容, 热扩散率, 热扩散速率, 热扩散速度, 导热率, 导热速率, 导热速度, 热通, 热参数, 热流, 导热系数, 灌注, 水分, 汗, 含水, 水量, 干燥, 湿度, 信噪比, 质量, 加热, 升温, 降温, 冷却, heat conductivity, thermal conductivity, heat diffusivity, thermal diffusivity, heat capacity, thermal capacity, capacity of heat, capacity of thermal, diffusivity of heat, diffusivity of thermal, flow of heat, heat flow, heat flux, flux of heat, thermal flux, thermal flow, flow of thermal, flux of thermal, perfusion, moisture, sweat, dry, humidity, signal to noise ratio, signal-to-noise ratio, noise-signal ratio, SNR, quality, heat+, cool+

### C. DOCUMENTS CONSIDERED TO BE RELEVANT

| Category* | Citation of document, with indication, where appropriate, of the relevant passages | Relevant to claim No. |
|---|---|---|
| Y | CN 114947765 A (HUAWEI TECHNOLOGIES CO., LTD.) 30 August 2022 (2022-08-30) description, paragraphs [0066]-[0192], and figures 1-11 | 1-26 |
| Y | US 2019126721 A1 (JAGUAR LAND ROVER LTD.) 02 May 2019 (2019-05-02) description, paragraphs [0035]-[0039] | 1-26 |
| Y | US 2020037884 A1 (NEC CORP. et al.) 06 February 2020 (2020-02-06) description, paragraphs [0061]-[0131] | 10, 24-26 |
| Y | CN 113164091 A (MICROSOFT TECHNOLOGY LICENSING LLC.) 23 July 2021 (2021-07-23) description, paragraphs [0015]-[0064], and figures 1-9 | 1-26 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **29 January 2024** | **15 February 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/136092** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 101346099 A (KONINKL PHILIPS ELECTRONICS N.V.) 14 January 2009 (2009-01-14)<br>    entire document | 1-26 |
| A | US 2004039271 A1 (BLANK THOMAS B et al.) 26 February 2004 (2004-02-26)<br>    entire document | 1-26 |

Form PCT/ISA/210 (second sheet) (July 2022)

**INTERNATIONAL SEARCH REPORT**
Information on patent family members

International application No.

**PCT/CN2023/136092**

| Patent document cited in search report | | | Publication date (day/month/year) | Patent family member(s) | | | Publication date (day/month/year) |
|---|---|---|---|---|---|---|---|
| CN | 114947765 | A | 30 August 2022 | None | | | |
| US | 2019126721 | A1 | 02 May 2019 | US | 10926605 | B2 | 23 February 2021 |
| US | 2020037884 | A1 | 06 February 2020 | US | 11744469 | B2 | 05 September 2023 |
| CN | 113164091 | A | 23 July 2021 | None | | | |
| CN | 101346099 | A | 14 January 2009 | CN | 101346099 | B | 05 October 2011 |
| US | 2004039271 | A1 | 26 February 2004 | US | 2008200783 | A9 | 21 August 2008 |
| | | | | US | 7509153 | B2 | 24 March 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211549724